(19) Europäisches Patentamt
European Patent Office
Office européen des brevets

(11) EP 2 040 700 B1

(12) **EUROPEAN PATENT SPECIFICATION**

(45) Date of publication and mention of the grant of the patent:
**24.11.2010 Bulletin 2010/47**

(21) Application number: **07729898.2**

(22) Date of filing: **05.06.2007**

(51) Int Cl.:
***A61K 31/415*** *(2006.01)* ***A61P 25/04*** *(2006.01)*
***A61P 25/28*** *(2006.01)*

(86) International application number:
**PCT/EP2007/055518**

(87) International publication number:
**WO 2007/141267 (13.12.2007 Gazette 2007/50)**

(54) **N- (PHENYLMETHYL) -2- (1H-PYRAZ0L-4-YL) ACETAMIDE DERIVATIVES AS P2X7 ANTAGONISTS FOR THE TREATMENT OF PAIN, INFLAMMATION AND NEURODEGENERATION**

N- (PHENYLMETHYL) -2- (1H-PYRAZOL-4-YL) ACETAMID-DERIVATE ALS P2X7-ANTAGONISTEN ZUR BEHANDLUNG VON SCHMERZEN, ENTZÜNDUNGEN UND NEURODEGENERATION

DÉRIVÉS DE N- (PHÉNYLMÉTHYL) -2- (1H-PYRAZ0L-4-YL) ACÉTAMIDE UTILISÉS COMME ANTAGONISTES FP2X7 POUR LE TRAITEMENT DE LA DOULEUR, DE L'INFLAMMATION ET DE LA NEURODÉGÉNÉRESCENCE

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**HR**

(30) Priority: **06.06.2006 GB 0611155**
**26.03.2007 GB 0705802**

(43) Date of publication of application:
**01.04.2009 Bulletin 2009/14**

(73) Proprietor: **Glaxo Group Limited**
**Greenford**
**Middlesex**
**UB6 0NN (GB)**

(72) Inventors:
- **BESWICK, Paul, John**
**08960 Sant Just Desvern**
**Barcelona (ES)**
- **CHAMBERS, Laura, J.**
**Harlow Essex CM19 5AW (GB)**
- **DAVIES, David, John**
**Stevenage Hertfordshire SG1 2NY (GB)**
- **DEAN, David, Kenneth**
**Harlow Essex CM19 5AW (GB)**
- **DEMONT, Emmanuel, Hubert**
**Harlow Essex CM19 5AW (GB)**
- **ROOMANS, Susan**
**Harlow Essex CM19 5AW (GB)**
- **WALTER, Daryl, Simon**
**Harlow Essex CM19 5AW (GB)**

(74) Representative: **Breen, Anthony Paul**
**GlaxoSmithKline**
**Corporate Intellectual Property (CN9.25.1)**
**980 Great West Road**
**Brentford, Middlesex TW8 9GS (GB)**

(56) References cited:
**WO-A-2005/019182**

EP 2 040 700 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to heterocyclic amide derivatives which modulate P2X7 receptor function and are capable of antagonizing the effects of ATP at the P2X7 receptor (P2X7 receptor antagonists); to processes for their preparation; to pharmaceutical compositions containing them; and to the use of such compounds in therapy.

The P2X7 receptor is a ligand-gated ion-channel which is expressed in cells of the hematopoietic lineage, e.g. macrophages, microglia, mast cells, and lymphocytes (T and B) (see, for example, Collo, et al. Neuropharmacology, Vol.36, pp1277-1283 (1997)), and is activated by extracellular nucleotides, particularly adenosine triphosphate (ATP). Activation of P2X7 receptors has been implicated in giant cell formation, degranulation, cytolytic cell death, CD62L shedding, regulation of cell proliferation, and release of proinflammatory cytokines such as interleukin 1 beta (IL-1β) (e.g. Ferrari, et al., J. Immunol., Vol. 176, pp3877-3883 (2006)) and tumour necrosis factor alpha (TNFα) (e.g. Hide, et al. Journal of Neurochemistry, Vol.75, pp965-972 (2000)). P2X7 receptors are also located on antigen presenting cells, keratinocytes, parotid cells, hepatocytes, erythrocytes, erythroleukaemic cells, monocytes, fibroblasts, bone marrow cells, neurones, and renal mesangial cells. Furthermore, the P2X7 receptor is expressed by presynaptic terminals in the central and peripheral nervous systems and has been shown to mediate glutamate release in glial cells (Anderson, C. et al. Drug. Dev. Res., Vol.50, page 92 (2000)).

The localisation of the P2X7 receptor to key cells of the immune system, coupled with its ability to release important inflammatory mediators from these cells suggests a potential role of P2X7 receptor antagonists in the treatment of a wide range of diseases including pain and neurodegenerative disorders. Recent preclinical *in vivo* studies have directly implicated the P2X7 receptor in both inflammatory and neuropathic pain (Dell'Antonio et al., Neurosci. Lett., Vol. 327, pp87-90 (2002),. Chessell, IP., et al., Pain, Vol.114, pp386-396 (2005), Honore et al., J. Pharmacol. Exp. Ther., Vol.319, p1376-1385 (2006)) while there is in vitro evidence that P2X7 receptors mediate microglial cell induced death of cortical neurons (Skaper, S.D., et al.,Glia, Vol.54, p234-242 (2006)). In addition, up-regulation of the P2X7 receptor has been observed around β-amyloid plaques in a transgenic mouse model of Alzheimer's disease (Parvathenani, L. et al. J. Biol. Chem., Vol.278(15), pp13309-13317 (2003)).

WO 2005/019182 (Bayer Healthcare AG) describes pyrazol derivatives with a P2X7 antagonistic activity that are substituted at the 4-position with a methylbenzamide group.

The present invention provides compounds which modulate P2X7 receptor function and are capable of antagonizing the effects of ATP at the P2X7 receptor (P2X7 receptor antagonists). In a first aspect, a compound of formula (I), or a pharmaceutically acceptable salt thereof, is provided:

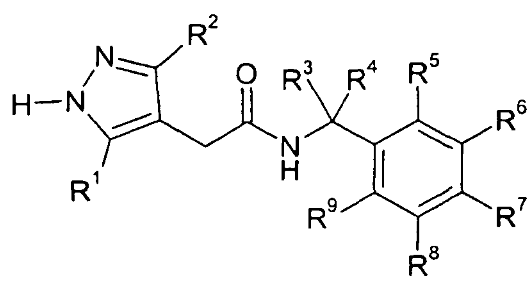

(I)

wherein:

- $R^1$ and $R^2$ represent $C_{1-6}$ alkyl, phenyl, or a $C_{3-6}$ cycloalkyl, any of which is optionally substituted with 1, 2 or 3 halogen atoms;
- $R^3$ and $R^4$ independently represent hydrogen or $C_{1-3}$ alkyl;
- $R^5$, $R^6$, $R^7$, $R^8$ and $R^9$ independently represent hydrogen, halogen, cyano, $C_{1-6}$ alkyl, $C_{2-6}$ alkenyl, $C_{2-6}$ alkynyl, $C_{3-6}$ cycloalkyl or phenyl, and any of said $C_{1-6}$ alkyl, $C_{2-6}$ alkenyl, $C_{2-6}$ alkynyl, $C_{3-6}$ cycloalkyl or phenyl is optionally substituted with 1, 2 or 3 halogen atoms; or $R^8$ and $R^9$ together with the carbon atoms to which they are attached form a benzene ring which is optionally substituted with 1, 2 or 3 halogen atoms;
- or $R^4$ and $R^5$ together with the carbon atoms to which they are attached form a $C_{5-7}$ cycloalkyl;
- with the proviso that when $R^5$ and $R^9$ are both selected from hydrogen or fluorine, at least one of $R^6$, $R^7$ and $R^8$ is a halogen atom, or $R^6$, $R^7$ and $R^8$ are selected from the group consisting of hydrogen, methyl and $CF_3$ and one, but not more than one, of $R^6$, $R^7$ and $R^8$ is methyl or $CF_3$.

In one embodiment of the invention, the compound of formula (I) is other than N-[1-(4-bromophenyl)propyl]-2-(3,5-dimethyl-1*H*-pyrazol-4-yl)acetamide.

In one embodiment of the invention, a compound of formula (IA), or a pharmaceutically acceptable salt thereof is provided:

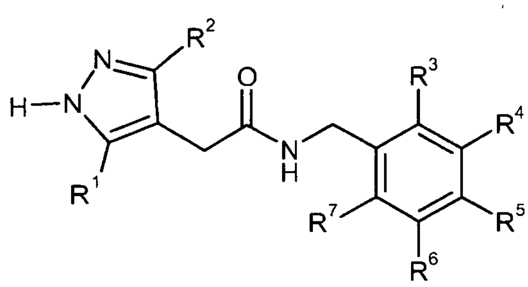

(IA)

wherein:

$R^1$ and $R^2$ represent $C_{1-6}$ alkyl, phenyl, or a $C_{3-6}$ cycloalkyl, any of which is optionally substituted with 1, 2 or 3 halogen atoms;
$R^3$, $R^4$, $R^5$, $R^6$ and $R^7$ independently represent hydrogen, halogen, cyano, $C_{1-6}$ alkyl, $C_{2-6}$ alkenyl, $C_{2-6}$ alkynyl, $C_{3-6}$ cycloalkyl or phenyl, and any of said $C_{1-6}$ alkyl, $C_{2-6}$ alkenyl, $C_{2-6}$ alkynyl, $C_{3-6}$ cycloalkyl or phenyl is optionally substituted with 1, 2 or 3 halogen atoms; or $R^6$ and $R^7$ together with the carbon atoms to which they are attached form a benzene ring which is optionally substituted with 1, 2 or 3 halogen atoms;
with the proviso that when $R^3$ and $R^7$ are both selected from hydrogen or fluorine, at least one of $R^4$, $R^5$ and $R^6$ is a halogen atom, or not more than one of $R^4$, $R^5$ and $R^6$ is a $CF_3$ group.

In one embodiment of the invention, a compound of formula (IB), or a pharmaceutically acceptable salt thereof is provided:

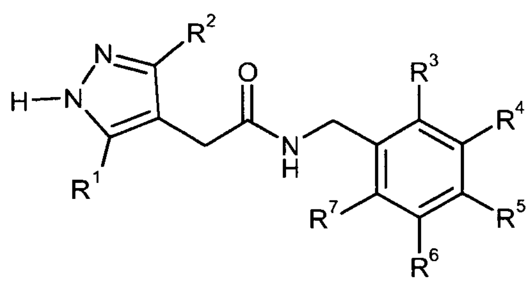

(IB)

wherein:

$R^1$ and $R^2$ represent $C_{1-6}$ alkyl, phenyl, or a $C_{3-6}$ cycloalkyl, any of which may be optionally substituted with 1, 2 or 3 halogen atoms;
$R^3$, $R^4$, $R^5$, $R^6$ and $R^7$ independently represent hydrogen, halogen, cyano, $C_{1-6}$ alkyl, $C_{2-6}$ alkenyl, $C_{2-6}$ alkynyl, $C_{3-6}$ cycloalkyl, or phenyl; and any of said $C_{1-6}$ alkyl, $C_{2-6}$ alkenyl, $C_{2-6}$ alkynyl, $C_{3-6}$ cycloalkyl, or phenyl may be optionally substituted with 1, 2 or 3 halogen atoms;
with the proviso that when $R^3$ and $R^7$ both represent hydrogen, at least one of $R^4$, $R^5$ and $R^6$ is a halogen atom.

As used herein, the term "alkyl" (when used as a group or as part of a group) refers to a straight or branched hydrocarbon chain containing the specified number of carbon atoms. For example, $C_{1-6}$ alkyl means a straight or branched hydrocarbon chain containing at least 1 and at most 6 carbon atoms. Examples of alkyl include, but are not limited to; methyl (Me), ethyl (Et), n-propyl, i-propyl, n-hexyl and i-hexyl.

As used herein, the term "alkenyl" refers to a straight or branched hydrocarbon chain containing the specified number of carbon atoms wherein at least once carbon-carbon bond is a double bond. Examples of alkenyl include, but are not limited to ethenyl, propenyl, n-butenyl, i-butenyl, n-pentenyl and i-pentenyl.

As used herein, the term "alkynyl" refers to a straight or branched hydrocarbon chain containing the specified number of carbon atoms wherein at least once carbon-carbon bond is a triple bond. Examples of alkynyl include, but are not limited to ethynyl, propynyl, butynyl, i-pentynyl, n-pentynyl, i-hexynyl and n-hexynyl.

The term 'cycloalkyl' unless otherwise stated means a closed 3 to 8 membered nonaromatic ring, for example cyclobutyl, cyclopentyl, cyclohexyl, cycloheptyl or cyclooctyl.

The term 'halogen' is used herein to describe, unless otherwise stated, a group selected from fluorine, chlorine, bromine or iodine.

In certain embodiments of the invention, $R^1$ and $R^2$ independently represent unsubstituted $C_{1-6}$ alkyl, trifluoromethyl, phenyl or a $C_{3-6}$ cycloalkyl. In one embodiment, $R^1$ and $R^2$ independently represent unsubstituted $C_{1-6}$ alkyl or trifluoromethyl. In another embodiment, $R^1$ and $R^2$ independently represent methyl or trifluoromethyl. In a further embodiment, $R^1$ represents trifluoromethyl and $R^2$ represents methyl.

In certain embodiments of the invention, $R^3$ and $R^4$ independently represent hydrogen or methyl. In one embodiment, $R^3$ and $R^4$ both represent hydrogen.

In certain embodiments of the invention, $R^5$, $R^6$, $R^7$, $R^8$ and $R^9$ independently represent hydrogen, halogen, cyano, trifluoromethyl or unsubstituted $C_{1-6}$ alkyl. In a further embodiment, $R^5$, $R^6$, $R^7$, $R^8$ and $R^9$ independently represent hydrogen, halogen, cyano, methyl or trifluoromethyl. In one embodiment, $R^5$, $R^6$, $R^7$, $R^8$ and $R^9$ independently represent hydrogen, chlorine, fluorine, bromine, methyl or trifluoromethyl.

In one embodiment of the invention there is provided a compound of formula (I), or a pharmaceutically acceptable salt thereof, wherein:

$R^1$ and $R^2$ independently represent unsubstituted $C_{1-6}$ alkyl, trifluoromethyl, phenyl or a $C_{3-6}$ cycloalkyl;
$R^3$ and $R^4$ independently represent hydrogen or methyl;
$R^5$, $R^6$, $R^7$, $R^8$ and $R^9$ independently represent hydrogen, halogen, cyano, trifluoromethyl or unsubstituted $C_{1-6}$ alkyl;
or $R^4$ and $R^5$ together with the carbon atoms to which they are attached form a $C_{5-7}$ cycloalkyl;
with the proviso that when $R^5$ and $R^9$ are both selected from hydrogen or fluorine, at least one of $R^6$, $R^7$ and $R^8$ is a halogen atom, or $R^6$, $R^7$ and $R^8$ are selected from the group consisting of hydrogen, methyl and $CF_3$ and one, but not more than one, of $R^6$, $R^7$ and $R^8$ is methyl or $CF_3$.

In a further embodiment, there is provided a compound of formula (I), or a pharmaceutically acceptable salt thereof, wherein:

$R^1$ and $R^2$ independently represent methyl or trifluoromethyl;
$R^3$ and $R^4$ both represent hydrogen; and
$R^5$, $R^6$, $R^7$, $R^8$ and $R^9$ independently represent hydrogen, halogen, trifluoromethyl or methyl;
with the proviso that when $R^5$ and $R^9$ are both selected from hydrogen or fluorine, at least one of $R^6$, $R^7$ and $R^8$ is a halogen atom, or $R^6$, $R^7$ and $R^8$ are selected from the group consisting of hydrogen, methyl and $CF_3$ and one, but not more than one, of $R^6$, $R^7$ and $R^8$ is methyl or $CF_3$.

Particular compounds according to the invention include the compounds of Examples 1-37 as shown below, or a pharmaceutically acceptable salt thereof.

Antagonists of P2X7 may be useful in preventing, treating, or ameliorating a variety of pain states (e.g. neuropathic pain, chronic inflammatory pain, and visceral pain), inflammation and neurodegeneration, in particular Alzheimer's disease. P2X7 antagonists may also constitute useful therapeutic agents in the management of rheumatoid arthritis and inflammatory bowel disease.

Compounds of the present invention which modulate P2X7 receptor function and are capable of antagonizing the effects of ATP at the P2X7 receptor (P2X7 receptor antagonists) may be competitive antagonists, inverse agonists, negative allosteric modulators or indirect modulators of receptor function.

Certain compounds of formula (I) may in some circumstances form acid addition salts thereof. It will be appreciated that for use in medicine compounds of formula (I) may be used as salts, in which case the salts should be pharmaceutically acceptable. Pharmaceutically acceptable salts include those described by Berge, Bighley and Monkhouse , J. Pharm. Sci., 1977, 66, 1-19. Basic compounds of formula (I) may form salts with pharmaceutically acceptable acids including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Examples of pharmaceutically acceptable salts include those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The compounds of formula (I) may be prepared in crystalline or non-crystalline form, and, if crystalline, may optionally be solvated, e.g. as the hydrate. This invention includes within its scope stoichiometric solvates (e.g. hydrates) as well as compounds containing variable amounts of solvent (e.g. water).

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The subject invention also includes isotopically-labeled compounds, which are identical to those recited in formula (I) and following, but for the fact that one or more atoms are replaced by an atom having an atomic mass or mass number different from the atomic mass or mass number most commonly found in nature. Examples of isotopes that can be incorporated into compounds of the invention include isotopes of hydrogen, carbon, nitrogen, oxygen, phosphorous, fluorine, iodine, and chlorine, such as 3H, 11C, 14C, 18F, 1231 and 1251.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as 3H, 14C are incorporated, are useful in drug and/or substrate tissue distribution assays. Tritiated, i.e., 3H, and carbon-14, i.e., 14C, isotopes are particularly preferred for their ease of preparation and detectability. 11C and 8F isotopes are particularly useful in PET (positron emission tomography), and 1251 isotopes are particularly useful in SPECT (single photon emission computerized tomography). PET and SPECT are useful in brain imaging. Further, substitution with heavier isotopes such as deuterium, i.e., 2H, can afford certain therapeutic advantages resulting from greater metabolic stability, for example increased in vivo half-life or reduced dosage requirements and, hence, may be preferred in some circumstances. Isotopically labeled compounds of formula (I) and following of this invention can generally be prepared by carrying out the procedures disclosed in the Schemes and/or in the Examples below, by substituting a readily available isotopically labeled reagent for a non-isotopically labeled reagent.

Preparation of compounds

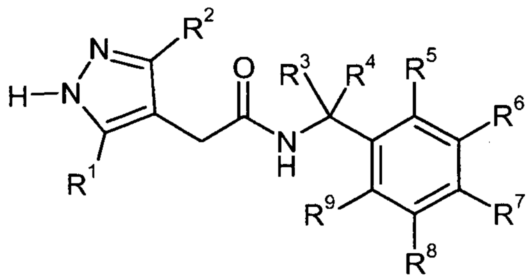

(I)

Compounds of formula (I), wherein the variables are defined above, and salts and solvates thereof may be prepared by the methodology described hereinafter, constituting a further aspect of this invention.

A process according to the invention for preparing a compound of formula (I) which comprises:

(a) Coupling of a carboxylic acid of formula (2) (or an activated derivative thereof) with an amine of formula (3) (see Scheme 1), wherein $R^1$, $R^2$, $R^3$, $R^4$, $R^5$, $R^6$, $R^7$, $R^8$, and $R^9$ are as defined above. Compounds (2) and (3) are optionally protected;

(b) Deprotecting a compound of formula (I) which is protected. Examples of protecting groups and the means for their removal can be found in T.W. Greene and P.G.M. Wuts Protective Groups in Organic Synthesis' (J.Wiley and

Sons, 3rd Ed. 1999); or

(c) Interconversion of compounds of formula (I) to other compounds of formula (I). Examples of conventional interconversion procedures include epimerisation, oxidation, reduction, alkylation, aromatic substitution, nucleophilic substitution, amide coupling and ester hydrolysis.

Scheme 1.

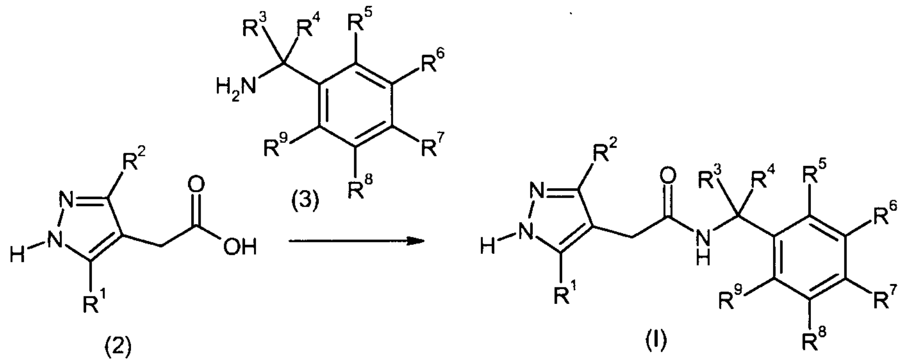

(2) (3) (I)

The coupling of an acid of formula (2) and an amine of formula (3) typically comprises the use of activating agents, such as water soluble carbodiimide or polymer-supported carbodiimide, 1-hydroxybenzotriazole (HOBT) or 1-hydroxy-7-azabenzotriazole (HOAt), and optionally a suitable base such as a tertiary alkylamine (e.g. diisopropylethylamine, N-ethyl morpholine, triethylamine) or pyridine, in a suitable solvent such as DMF and/or dichloromethane and at a suitable temperature e.g. between 0°C and room temperature. Alternatively the coupling of (2) and (3) may be accomplished by treatment with O-(7-azabenzotriazol-1-yl)-N,N,N',N'-tetramethyluronium hexafluorophosphate and a suitable tertiary alkylamine such as diisopropylethylamine in a suitable solvent such as dimethylformamide at a suitable temperature such as room temperature. Where the compound of formula (2) is an activated derivative (e.g. acid chloride, mixed anhydride, active ester (e.g. O-acyl-isourea)), process (a) typically comprises treatment of said activated derivative with an amine (Ogliaruso, M.A.; Wolfe, J.F. in The Chemistry of Functional Groups (Ed. Patai, S.) Suppl.B: The Chemistry of Acid Derivatives, Pt. 1 (John Wiley and Sons, 1979), pp442-8; Beckwith, A.L.J. in The Chemistry of Functional Groups (Ed. Patai, S.) Suppl.B: The Chemistry of Amides (Ed. Zabricky, J.) (John Wiley and Sons, 1970), pp 73 ff.

Representative methods for the preparation of compounds of formula (2) are shown in Schemes 2-3 below:

Scheme 2

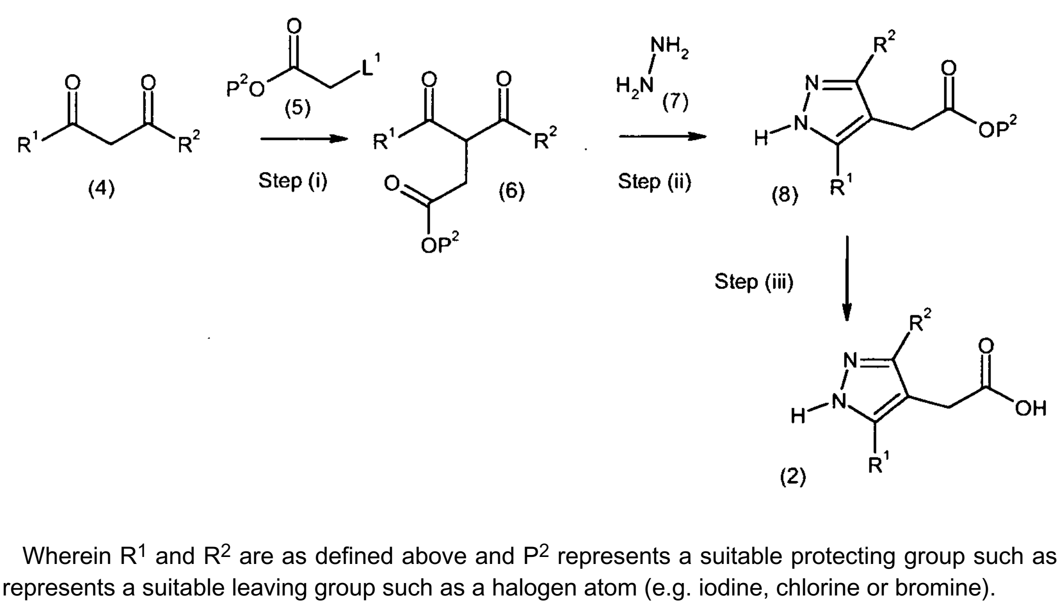


(4) (5) (6) (7) (8) (2)

Wherein $R^1$ and $R^2$ are as defined above and $P^2$ represents a suitable protecting group such as $C_{1-6}$ alkyl, and $L^1$ represents a suitable leaving group such as a halogen atom (e.g. iodine, chlorine or bromine).

An analogous process has been described previously (US 4,146,721).

Step (i) typically comprises the use of a suitable solvent such as a dioxane/water mixture or dimethyl formamide and a suitable base such as potassium hydroxide or sodium hydride at a suitable temperature e.g. between 0°C and room temperature. Step (ii) typically comprises the use of hydrazine (7) or a salt (e.g. HCl) thereof in a suitable solvent such as ethanol. The reaction mixtures are typically heated at a suitable temperature such as reflux temperature.

Step (iii) typically comprises a standard procedure for conversion of a carboxylic ester to an acid, such as use of an appropriate hydroxide salt (e.g. lithium hydroxide) in an appropriate solvent such as a mixture of tetrahydrofuran and water at a suitable temperature such as 50°C, or use of an appropriate acid (e.g. trifluoroacetic acid) in an appropriate solvent such as dichloromethane at a suitable temperature such as room temperature.

Scheme 3

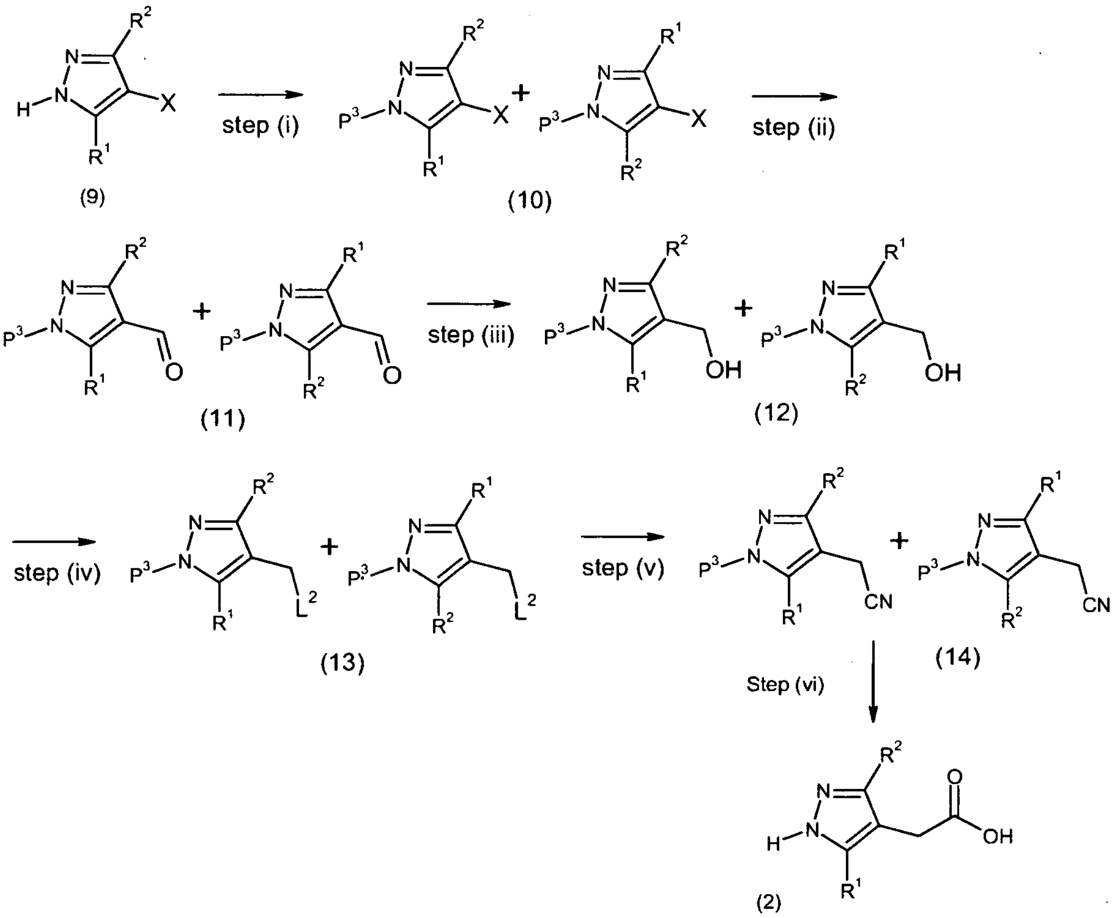


Wherein $R^1$ and $R^2$ are as defined above, $P^3$ represents a suitable protecting group such as 2-(trimethylsilyl) ethoxymethyl, X represents a suitable exchangeable group such as halogen (e.g. bromine or iodine) and $L^2$ represents a suitable leaving group such as a sulphonate ester (e.g. methanesulphonyl ester).

Step (i) typically comprises treatment with a suitable protecting group using a suitable reagent such as {2-[(chloromethyl)oxy]ethyl}(trimethyl)silane chloride in a suitable solvent such as a tetrahydrofuran with a suitable base such as sodium hydride at a suitable temperature such as 5°C.

Step (ii) typically comprises treatment of (10) with a suitable alkyl lithium species such as n-butyl lithium and a suitable carbonylating agent such as dimethylformamide in a suitable solvent such as tetrahydrofuran and at a suitable temperature such as between -78°C and room temperature.

Step (iii) typically comprises reduction of (11) using a suitable reducing agent such as sodium borohydride in a suitable solvent such as ethanol and at a suitable temperature such as between 0°C and room temperature.

Step (iv) typically comprises treatment of (12) with a suitable sulphonyl chloride such as methanesulphonyl chloride and a suitable base such as triethylamine in a suitable solvent such as dichloromethane and at a suitable

temperature such as between 0˚C and room temperature.

Step (v) typically comprises treatment of (13) with a suitable cyanide salt such as potassium cyanide in a suitable solvent such as dimethylsulphoxide and at a suitable temperature such as between room temperature and 80˚C.

Step (vi) typically comprises treatment of (14) with a suitable acid such as 5N hydrochloric acid in a suitable solvent such as 1,4-dioxane at a suitable temperature such as 100˚C.

Compounds of the general formulae (3), (4), (5), (7), and (9) are typically either available from commercial sources or can be prepared by a person skilled in the art using methods described in the chemical literature (or using analogous methods).

Pharmaceutically acceptable salts may be prepared conventionally by reaction with the appropriate acid or acid derivative.

Clinical Indications

It is believed that as compounds of the present invention modulate P2X7 receptor function and are capable of antagonizing the effects of ATP at the P2X7 receptor they may be useful in the treatment of pain, including acute pain, chronic pain, chronic articular pain, musculoskeletal pain, neuropathic pain, inflammatory pain, visceral pain, pain associated with cancer, pain associated with migraine, tension headache and cluster headaches, pain associated with functional bowel disorders, lower back and neck pain, pain associated with sprains and strains, sympathetically maintained pain; myositis, pain associated with influenza or other viral infections such as the common cold, pain associated with rheumatic fever, pain associated with myocardial ischemia, post operative pain, cancer chemotherapy, headache, toothache and dysmenorrhea.

Chronic articular pain conditions include rheumatoid arthritis, osteoarthritis, rheumatoid spondylitis, gouty arthritis and juvenile arthritis.

Pain associated with functional bowel disorders includes non-ulcer dyspepsia, non-cardiac chest pain and irritable bowel syndrome.

Neuropathic pain syndromes include: diabetic neuropathy, sciatica, non-specific lower back pain, trigeminal neuralgia, multiple sclerosis pain, fibromyalgia, HIV-related neuropathy, post-herpetic neuralgia, trigeminal neuralgia, and pain resulting from physical trauma, amputation, phantom limb syndrome, spinal surgery, cancer, toxins or chronic inflammatory conditions. In addition, neuropathic pain conditions include pain associated with normally non-painful sensations such as "pins and needles" (paraesthesias and dysesthesias), increased sensitivity to touch (hyperesthesia), painful sensation following innocuous stimulation (dynamic, static, thermal or cold allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

Other conditions which could potentially be treated by compounds of the present invention include fever, inflammation, immunological diseases, abnormal platelet function diseases (e.g. occlusive vascular diseases), impotence or erectile dysfunction; bone disease characterised by abnormal bone metabolism or resorbtion; hemodynamic side effects of non-steroidal anti-inflammatory drugs (NSAID's) and cyclooxygenase-2 (COX-2) inhibitors, cardiovascular diseases; neurodegenerative diseases and neurodegeneration, neurodegeneration following trauma, tinnitus, dependence on a dependence-inducing agent such as opiods (e.g. morphine), CNS depressants (e.g. ethanol), psychostimulants (e.g. cocaine) and nicotine; complications of Type I diabetes, kidney dysfunction, liver dysfunction (e.g. hepatitis, cirrhosis), gastrointestinal dysfunction (e.g. diarrhoea), colon cancer, overactive bladder and urge incontinence. Depression and alcoholism could potentially also be treated by compounds of the present invention.

Inflammation and the inflammatory conditions associated with said inflammation include skin conditions (e.g. sunburn, burns, eczema, dermatitis, allergic dermatitis, psoriasis), meningitis, ophthalmic diseases such as glaucoma, retinitis, retinopathies, uveitis and of acute injury to the eye tissue (e.g. conjunctivitis), inflammatory lung disorders (e.g. asthma, bronchitis, emphysema, allergic rhinitis, respiratory distress syndrome, pigeon fancier's disease, farmer's lung, chronic obstructive pulmonary disease (COPD), airways hyperresponsiveness); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastrointestinal reflux disease); organ transplantation and other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, gingivitis, myocardial ischemia, pyrexia, systemic lupus erythematosus, polymyositis, tendinitis, bursitis, and Sjogren's syndrome.

Immunological diseases include autoimmune diseases, immunological deficiency diseases or organ transplantation.

Bone diseases characterised by abnormal bone metabolism or resorbtion include osteoporosis (especially postmenopausal osteoporosis), hyper-calcemia, hyperparathyroidism, Paget's bone diseases, osteolysis, hypercalcemia of malignancy with or without bone metastases, rheumatoid arthritis, periodontitis, osteoarthritis, ostealgia, osteopenia, cancer cacchexia, calculosis, lithiasis (especially urolithiasis), solid carcinoma, gout and ankylosing spondylitis, tendinitis and bursitis. Cardiovascular diseases include hypertension or myocardiac ischemia; atherosclerosis; functional or organic venous insufficiency; varicose therapy; haemorrhoids; and shock states associated with a marked drop in arterial pressure (e.g. septic shock).

Neurodegenerative diseases include dementia, particularly degenerative dementia (including senile dementia, dementia with Lewy bodies, Alzheimer's disease, Pick's disease, Huntingdon's chorea, Parkinson's disease and Creutzfeldt-Jakob disease, ALS, motor neuron disease); vascular dementia (including multi-infarct dementia); as well as dementia associated with intracranial space occupying lesions; trauma; infections and related conditions (including HIV infection, meningitis and shingles); metabolism; toxins; anoxia and vitamin deficiency; and mild cognitive impairment associated with ageing, particularly Age Associated Memory Impairment.

The compounds of formula (I) may also be useful as neuroprotectants and in the treatment of neurodegeneration following trauma such as stroke, cardiac arrest, pulmonary bypass, traumatic brain injury, spinal cord injury or the like.

The compounds of the present invention may also be useful in the treatment of malignant cell growth and/or metastasis, and myoblastic leukaemia.

Complications of Type 1 diabetes include diabetic microangiopathy, diabetic retinopathy, diabetic nephropathy, macular degeneration, glaucoma, nephrotic syndrome, aplastic anaemia, uveitis, Kawasaki disease and sarcoidosis.

Kidney dysfunction includes nephritis, glomerulonephritis, particularly mesangial proliferative glomerulonephritis and nephritic syndrome.

It is to be understood that reference to treatment includes both treatment of established symptoms and prophylactic treatment, unless explicitly stated otherwise.

According to a further aspect of the invention, we therefore provide a compound of formula (I) or a pharmaceutically acceptable salt thereof for use in human or veterinary medicine.

According to another aspect of the invention, we provide a compound of formula (I) or a pharmaceutically acceptable salt thereof for use in the treatment of a condition which is mediated by P2X7 receptors.

According to a further aspect of the invention, we provide a compound of formula (I) or a pharmaceutically acceptable salt thereof for use in the treatment of a human or animal subject suffering from a condition which is mediated by P2X7 receptors.

According to a further aspect of the invention we provide a compound of formula (I) or a pharmaceutically acceptable salt thereof for use in the treatment of a human or animal subject suffering from pain, inflammation or a neurodegenerative disease.

According to a yet further aspect of the invention we provide a compound of formula (I) or a pharmaceutically acceptable salt thereof for use in the treatment of a human or animal subject suffering from inflammatory pain, neuropathic pain or visceral painf.

According to a further aspect of the invention we provide a compound of formula (I) or a pharmaceutically acceptable salt thereof for use in the treatment of a human subject, suffering from Alzheimer's disease .

According to another aspect of the invention, we provide the use of a compound of formula (I) or a pharmaceutically acceptable salt thereof for the manufacture of a medicament for the treatment of a condition which is mediated by the action of P2X7 receptors.

According to another aspect of the invention we provide the use of a compound of formula (I) or a pharmaceutically acceptable salt thereof for the manufacture of a medicament for the treatment or prevention of pain, inflammation or a neurodegenerative disease.

According to another aspect of the invention we provide the use of a compound of formula (I) or a pharmaceutically acceptable salt thereof for the manufacture of a medicament for the treatment or prevention of inflammatory pain, neuropathic pain or visceral pain.

In one aspect of the invention we provide the use of a compound of formula (I) or a pharmaceutically acceptable salt thereof for the manufacture of a medicament for the treatment or prevention of Alzheimer's disease.

In order to use a compound of formula (I) or a pharmaceutically acceptable salt thereof for the treatment of humans and other mammals it is normally formulated in accordance with standard pharmaceutical practice as a pharmaceutical composition. Therefore in another aspect of the invention there is provided a pharmaceutical composition comprising a compound of formula (I), or a pharmaceutically acceptable salt thereof, adapted for use in human or veterinary medicine.

In order to use the compounds of formula (I) in therapy, they will normally be formulated into a pharmaceutical composition in accordance with standard pharmaceutical practice. The present invention also provides a pharmaceutical composition, which comprises a compound of formula (I), or a pharmaceutically acceptable salt thereof, and optionally a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders,

injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

The following Descriptions and Examples illustrate the preparation of compounds of the invention.

Examples:

The general methods (a)-(c), along with the synthetic methods outlined in Schemes 1-3 above, for the preparation of compounds of the present invention are further illustrated by the following examples.

Example 1 *N*-[(2-Chloro-4-fluorophenyl)methyl]-2-[3-methyl-5-(2-methylpropyl)-1*H*-pyrazol-4-yl]acetamide (E1)

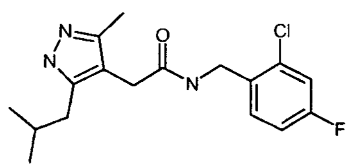

[3-Methyl-5-(2-methylpropyl)-1*H*-pyrazol-4-yl]acetic acid (0.170 g, 0.43 mmol, prepared as described below) was dissolved in a mixture of dimethylformamide (1 ml) and dichloromethane (3 ml) and and to this was added water soluble carbodiimide (0.099 g, 0.52 mmol), 1-hydroxybenzotriazole (0.070 g, 0.52 mmol), and N-ethyl morpholine (0.164 ml, 1.29 mmol). The mixture was stirred for 10 minutes and then [(2-chloro-4-fluorophenyl)methyl]amine (0.082 g, 0.52 mmol) was added. The mixture was stirred overnight at room temperature and then saturated aqueous sodium hydrogen carbonate (2 ml) was added to the mixture. After stirring for a further 10 minutes the organic phase was separated by filtration through a hydrophobic frit. The aqueous layer was washed with a further aliquot of dichloromethane (2-3 ml) and the organic phase was again separated and then the combined organic phases were evaporated to give the crude product as a yellow oil. The crude material was purified by mass-directed automated HPLC to give the pure product as a white solid after freeze-drying of the collected product fractions (0.080 g).
LC/MS $[M+H]^+$ = 338 retention time = 2.32 minutes.

The [3-methyl-5-(2-methylpropyl)-1*H*-pyrazol-4-yl]acetic acid used in the above procedure was obtained by hydrolysis of the corresponding ester (ethyl [3-methyl-5-(2-methylpropyl)-1*H*-pyrazol-4-yl]acetate). Ethyl [3-methyl-5-(2-methylpropyl)-1*H*-pyrazol-4-yl]acetate was in turn prepared by the reaction of ethyl 3-acetyl-6-methyl-4-oxoheptanoate (see preparative details below) with hydrazine hydrate. Appropriate methodologies to prepare these or analgous compounds have been described previously (*e.g.* US 4, 146, 721).

Ethyl 3-acetyl-6-methyl-4-oxoheptanoate was prepared in the following manner: 6-methyl-2,4-heptanedione (0.711 g, 5.0 mmol) was dissolved in dioxane (2 ml) and water (1 ml) and then cooled to 0˚C. To this was added, in a dropwise fashion, a solution of potassium hydroxide (0.281 g, 5.0 mmol) in water (2 ml). The mixture was then warmed to room temperature and stirred for 15 minutes. A solution of ethyl bromoacetate (0.554 ml, 5.0 mmol) in dioxane (1 ml) was then added dropwise and the mixture was stirred overnight at room temperature. The organic phase was separated and the aqueous phase was further extracted with ethyl acetate (2 x 10 ml). The combined organic fractions were dried over anhydrous sodium sulphate then filtered and evaporated to give a pale yellow oil. This material was purified by automated flash-silica column chromatography (Biotage SP4™), eluting with a gradient of 0-20% ethyl acetate in hexane, to give ethyl [3-methyl-5-(2-methylpropyl)-1*H*-pyrazol-4-yl]acetate (0.343 g) as a colourless oil.

Examples 2-3

In a manner analogous to that described for Example 1 above the compounds tabulated below (Table 1) were prepared by substituting the appropriate diketones for the 6-methyl-2,4-heptanedione used in the above procedure. All of the diketones required to prepare the examples listed in Table 1 are available from commercial sources or can be prepared using routes described previously in the chemical literature,

Table 1

| Example no. | Chemical name | $[M+H]^+$ | Retention time (mins) |
|---|---|---|---|
| E2 | 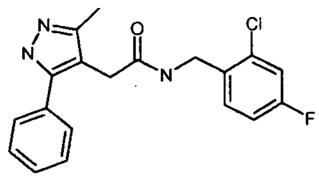 *N*-[(2-Chloro-4-fluorophenyl)methyl]-2-(3-methyl- 5-phenyl-1*H*-pyrazol-4-yl)acetamide | 358 | 2.56 |
| E3 | 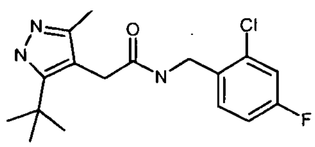 *N*-[(2-Chloro-4-fluorophenyl)methyl]-2-[5-(1,1- dimethylethyl)-3-methyl-1*H*-pyrazol-4- yl]acetamide | 338 | 2.32 |

Example 4 *N*-[(2-Chloro-4-fluorophenyl)methyl]-2-(3,5-dimethyl-1*H*-pyrazol-4-yl)acetamide (E4)

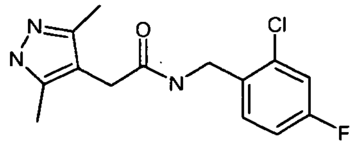

*N*-[(2-Chloro-4-fluorophenyl)methyl]-2-(3,5-dimethyl-1*H*-pyrazol-4-yl)acetamide was prepared in an analogous method to that described for the preparation of N-[(2-chloro-4-fluorophenyl)methyl]-2-[3-methyl-5-(2-methylpropyl)-1*H*-pyrazol-4-yl]acetamide (E1) but using (3,5-dimethyl-1*H*-pyrazol-4-yl)acetic acid in the place of [3-methyl-5-(2-methylpropyl)-1*H*-pyrazol-4-yl]acetic acid.
LC/MS $[M+H]^+$ = 296 retention time = 1.83 minutes.

Examples 5-29

In a manner analogous to that described for Example 4 above the compounds tabulated below (Table 2) were prepared by substituting the appropriate amines for the [(2-chloro-4-fluorophenyl)methyl]amine used in the above procedure. All of the amines required to prepare the examples listed inTable 2 are available from commercial sources or can be prepared using routes described previously in the chemical literature,

Table 2

| Example no. | Chemical name | $[M+H]^+$ | Retention time (mins) |
|---|---|---|---|
| E5 | 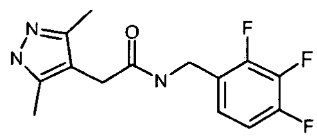 2-(3,5-Dimethyl-1*H*-pyrazol-4-yl)-*N*-[(2,3,4-trifluorophenyl) methyl]acetamide | 298 | 1.90 |
| E6 | 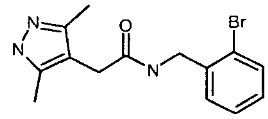 *N*-[(2-Bromophenyl)methyl]-2-(3,5-dimethyl-1*H*-pyrazol-4-yl) acetamide | 322 | 1.91 |
| E7 | 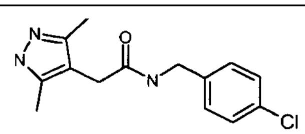 *N*-[(4-Chlorophenyl)methyl]-2-(3,5-dimethyl-1*H*-pyrazol-4-yl) acetamide | 278 | 1.93 |
| E8 | 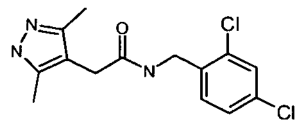 *N*-[(2,4-Dichlorophenyl)methyl]-2-(3,5-dimethyl-1*H*-pyrazol-4-yl)acetamide | 312 | 2.12 |
| E9 | 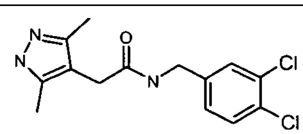 *N*-[(3,4-Dichlorophenyl)methyl]-2-(3,5-dimethyl-1*H*-pyrazol-4-yl)acetamide | 312 | 2.13 |
| E10 | 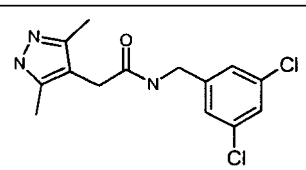 *N*-[(3,5-Dichlorophenyl)methyl]-2-(3,5-dimethy-1*H*-pyrazol-4-yl)acetamide | 312 | 2.14 |
| E11 | 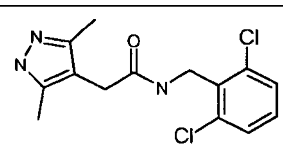 *N*-[(2,6-Dichlorophenyl)methyl]-2-(3,5-dimethyl-1*H*-pyrazol-4-yl)acetamide | 312 | 1.95 |

(continued)

| Example no. | Chemical name | $[M+H]^+$ | Retention time (mins) |
|---|---|---|---|
| E12 | 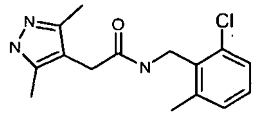 *N*-[(2-Chloro-6-methylphenyl)methyl]-2-(3,5-dimethyl-1*H*-pyrazol-4-yl)acetamide | 292 | 1.99 |
| E13 | 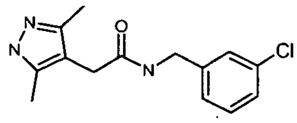 *N*-[(3-Chlorophenyl)methyl]-2-(3,5-dimethyl-1*H*-pyrazol-4-yl) acetamide | 278 | 1.91 |
| E14 | 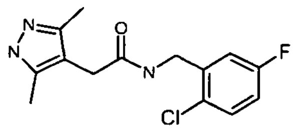 *N*-[(2-Chloro-5-fluorophenyl)methyl]-2-(3,5-dimethyl-1*H*-pyrazol-4-yl)acetamide | 297 | 1.85 |
| E15 | 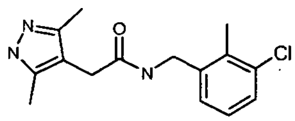 *N*-[(3-Chloro-2-methylphenyl)methyl]-2-(3,5-dimethyl-1*H*-pyrazol-4-yl)acetamide | 292 | 2.10 |
| E16 | 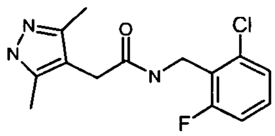 *N*-[(2-Chloro-6-fluorophenyl)methyl]-2-(3,5-dimethyl-1*H*-pyrazol-4-yl)acetamide | 296 | 1.82 |
| E17 | 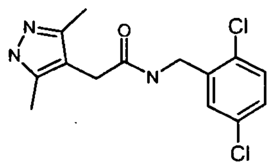 *N*-[(2,5-Dichlorophenyl)methyl]-2-(3,5-dimethyl-1*H*-pyrazol-4-yl)acetamide | 312 | 2.01 |
| E18 | 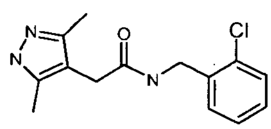 *N*-[(2-Chlorophenyl)methyl]-2-(3,5-dimethyl-1*H*-pyrazol-4-yl) acetamide | 278 | 1.87 |

(continued)

| Example no. | Chemical name | [M+H]+ | Retention time (mins) |
|---|---|---|---|
| E19 | 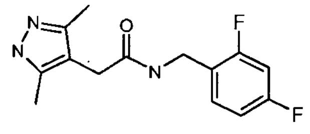 *N*-[(2,4-Difluorophenyl)methyl]-2-(3,5-dimethyl-1*H*-pyrazol-4-yl)acetamide | 280 | 1.75 |
| E20 | 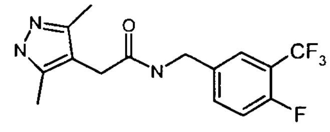 2-(3,5-Dimethyl-1*H*-pyrazol-4-yl)-*N*-{[4-fluoro-3-(trifluoromethyl)phenyl]methyl}acetamide | 330 | 2.14 |
| E21 | 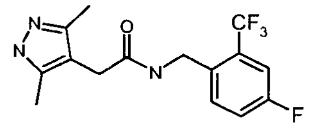 2-(3,5-Dimethyl-1*H*-pyrazol-4-yl)-*N*-{[4-fluoro-2-(trifluoromethyl)phenyl]methyl}acetamide | 330 | 2.03 |
| E22 | 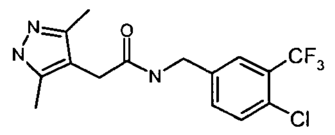 *N*-{[4-Chloro-3-(trifluoromethyl)phenyl]methyl}-2-(3,5-dimethyl-1*H*-pyrazol-4-yl)acetamide | 346 | 2.33 |
| E23 | 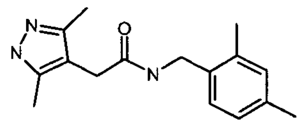 *N*-[(2,4-Dimethylphenyl)methyl]-2-(3,5-dimethyl-1*H*-pyrazol-4-yl)acetamide | 272 | 1.99 |
| E24 | 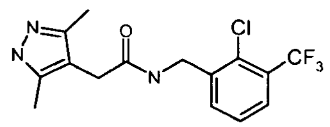 *N*-{[2-Chloro-3-(trifluoromethyl)phenyl]methyl}-2-(3,5-dimethyl-1*H*-pyrazol-4-yl)acetamide | 346 | 2.24 |
| E25 | 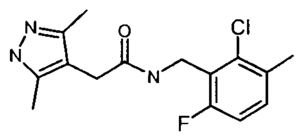 *N*-[(2-Chloro-6-fluoro-3-methylphenyl)methyl]-2-(3,5-dimethyl-1*H*-pyrazol-4-yl)acetamide | 310 | 2.00 |

(continued)

| Example no. | Chemical name | [M+H]+ | Retention time (mins) |
|---|---|---|---|
| E26 | 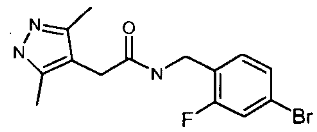 *N*-[(4-Bromo-2-fluorophenyl)methyl]-2-(3,5-dimethyl-1*H*-pyrazol-4-yl)acetamide | 340/342 | 2.02 |
| E27 | 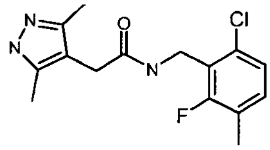 *N*-[(6-Chloro-2-fluoro-3-methylphenyl)methyl]-2-(3,5-dimethyl-1*H*-pyrazol-4-yl)acetamide | 310 | 2.04 |
| E28 | 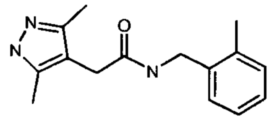 2-(3,5-Dimethyl-1*H*-pyrazol-4-yl)-*N*-[(2-methylphenyl)methyl] acetamide | 258 | 1.79 |
| E29 | 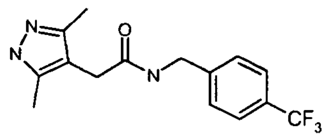 2-(3,5-Dimethyl-1*H*-pyrazol-4-yl)-*N*-{[4-(trifluoromethyl)phenyl] methyl}acetamide | 312 | 2.07 |

Example 30 *N*-[(2-Chloro-4-fluorophenyl)methyl]-2-(3,5-diethyl-1*H*-pyrazol-4-yl)acetamide (E30)

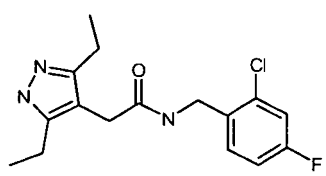

(3,5-Diethyl-1*H*-pyrazol-4-yl)acetic acid (0.260 g, ~1 mmol), prepared as described below) was dissolved in a mixture of dimethylformamide (0.5 ml) and dichloromethane (5 ml) and to this was added water soluble carbodiimide (0.230 g, 1.2 mmol), 1-hydroxybenzotriazole (0.162 g, 1.2 mmol), and N-ethyl morpholine (0.458 ml, 3.6 mmol). The mixture was stirred for 10 minutes and then [(2-chloro-4-fluorophenyl)methyl]amine (0.239 g, 1.5 mmol) was added. The mixture was stirred for -22 hrs at room temperature and then saturated aqueous sodium hydrogen carbonate (3 ml) was added to the mixture. After stirring for a further 10 minutes the organic phase was separated by filtration through a hydrophobic frit. The aqueous layer was washed with a further aliquot of dichloromethane (~1 ml) and the organic phase was again separated and then the combined organic phases were evaporated to give the crude product as an orange oil. The crude material was purified by mass-directed automated HPLC to give the pure product as an off-white solid after freeze-drying of the collected product fractions (0.049 g).
LC/MS $[M+H]^+$ = 324 retention time = 2.08 minutes.

The (3,5-Diethyl-1*H*-pyrazol-4-yl)acetic acid used in the above method was prepared as follows:

(i) 3,5-Heptanedione (2.03 ml, 15.0 mmol) was dissolved in dimethylformamide (10 ml) and cooled to 0˚C using an ice-bath. Sodium hydride (60% in oil, 0.630 g, 15.75 mmol) was then added slowly in portions and then the mixture was allowed to warm to room temperature and stirred for - 30 minutes. The mixture was then treated with *t*-butyl bromoacetate (2.21 ml, 15.0 mmol) and stirred overnight (~18 hrs) at room temperature. The mixture was concentrated, azeotroping with toluene to remove as much dimethylformamide as possible, and the residue was partitioned between water (~20 ml) and ethyl acetate (~20 ml). The organic layer was separated and the aqueous phase was acidified to -pH5 using 2N aqueous hydrogen chloride then further extracted with ethyl acetate (2 x 20 ml). The combined organic extracts were dried over sodium sulphate, filtered and concentrated to give a yellow oil. The oil was purified by automated (Biotage SP4™) flash-silica gel column chromatography, eluting with a 0-10% gradient of ethyl acetate in hexane, to give pure 1,1-dimethylethyl 4-oxo-3-propanoylhexanoate (2.15 g) as a yellow oil.

(ii) 1,1-Dimethylethyl 4-oxo-3-propanoylhexanoate (1.08 g, 4.46 mmol) was dissolved in ethanol (15 ml) and treated with hydrazine hydrate (0.857 ml, 17.66 mmol). The mixture was heated to reflux at 95˚C for 6 hrs then cooled to room temperature and left to stand over a weekend. Most of the ethanol was removed *in vacuo* and the remaining residue was partitioned between dichloromethane (10 ml) and water (5 ml). The organic layer was separated using a hydrophobic frit and the aqueous layer was further extracted with dichloromethane (2 x 10 ml). The combined organic extracts were concentrated to give 1,1-dimethylethyl (3,5-diethyl-1*H*-pyrazol-4-yl)acetate (1.08 g) as a pale yellow oil which was used in the next step without further purification.

(iii) 1,1-dimethylethyl (3,5-diethyl-1*H*-pyrazol-4-yl)acetate (1.07 g, 4.49 mmol) was dissolved in dichloromethane (~2 ml) and treated with trifluoroacetic acid (2 ml). The mixture was stirred at room temperature for 3.5 hrs and then evaporated *in vacuo*, azeotroping with toluene to remove traces of trifluoroacetic acid, to give crude (3,5-diethyl-1*H*-pyrazol-4-yl)acetic acid (1.52 g) as a dark orange/brown oil which was used without any further purification.

<u>Example 31</u> 2-(3,5-Diethyl-1*H*-pyrazol-4-yl)-*N*-[(2,3,4-trifluorophenyl)methyl]acetamide (E31)

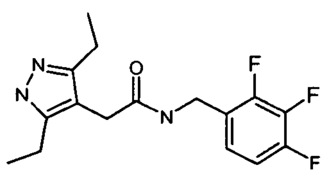

2-(3,5-Diethyl-1*H*-pyrazol-4-yl)-*N*-[(2,3,4-trifluorophenyl)methyl]acetamide was prepared in a manner analogous to that described for the synthesis of *N*-[(2-chloro-4-fluorophenyl)methyl]-2-(3,5-diethyl-1*H*-pyrazol-4-yl)acetamide (example 30) above but using [(2,3,4-trifluorophenyl)methyl]amine in the place of [(2-chloro-4-fluorophenyl)methyl]amine.
LC/MS $[M+H]^+$ = 326 retention time = 2.04 minutes.

<u>Example 32</u> 2-[3,5-Bis(1-methylethyl)-1*H*-pyrazol-4-yl]-*N*-[(2-chloro-4-fluorophenyl)methyl]acetamide (E32)

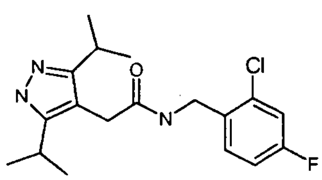

2-[3,5-Bis(1-methylethyl)-1*H*-pyrazol-4-yl]-*N*-[(2-chloro-4-fluorophenyl)methyl]acetamide was prepared in a manner analogous to that described for the synthesis of *N*-[(2-chloro-4-fluorophenyl)methyl]-2-(3,5-diethyl-1*H*-pyrazol-4-yl) acetamide (example 30) above but using 2,6-dimethyl-3,5-heptanedione in the place of 3,5-heptanedione.
LC/MS $[M+H]^+$ = 352 retention time = 2.35 minutes.

Example 33 2-[3,5-Bis(1-methylethyl)-1*H*-pyrazol-4-yl]-*N*-[(2,3,4-trifluorophenyl)methyl]acetamide (E33)

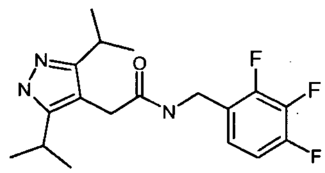

2-[3,5-Bis(1-methylethyl)-1*H*-pyrazol-4-yl]-*N*-[(2,3,4-trifluorophenyl)methyl]acetamide was prepared in a manner analogous to that described for the synthesis of *N*-[(2-chloro-4-fluorophenyl)methyl]-2-(3,5-diethyl-1*H*-pyrazol-4-yl)acetamide (example 30) above but using 2,6-dimethyl-3,5-heptanedione in the place of 3,5-heptanedione and using [(2,3,4-trifluorophenyl)methyl]amine in the place of [(2-chloro-4-fluorophenyl)methyl]amine.
LC/MS $[M+H]^+$ = 354 retention time = 2.31 minutes.

Example 34 *N*-[(2-Chloro-4-fluorophenyl)methyl]-2-[3-methyl-5-(trifluoromethyl)-1*H*-pyrazol-4-yl]acetamide (E34)

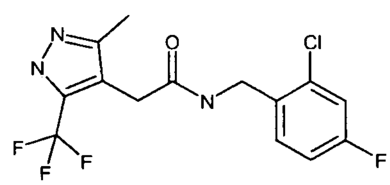

A mixture of crude [3-methyl-5-(trifluoromethyl)-1*H*-pyrazol-4-yl]acetic acid (0.187 g, 0.9 mmol, prepared as described below) in dichloromethane (10 ml) was treated with 1-hydroxybenzotriazole hydrate (0.149 g, 1.1 mmol), *N*-(3-dimethylaminopropyl)-N'-ethylcarbodiimide hydrochloride (0.211 g, 1.1 mmol) and *N*-ethyl morpholine (0.35 ml, 2.7 mmol) and the reaction mixture was stirred at room temperature for 15 minutes. [(2-Chloro-4-fluorophenyl)methyl]amine (0.128 g, 0.8 mmol) was added and the reaction mixture was stirred at room temperature for 16 hours. The mixture was diluted with dichloromethane and the organic solution was washed sequentially with saturated aqueous sodium hydrogen carbonate, water, citric acid, water and brine, dried and evaporated. The residue was purified by mass-directed automated HPLC and the resulting solid was triturated with ether/hexane, collected and dried to give *N*-[(2-chloro-4-fluorophenyl) methyl]-2-[3-methyl-5-(trifluoromethyl)-1*H*-pyrazol-4-yl]acetamide (E34)
LC/MS $[M+H]^+$ = 350, retention time = 2.46 minutes.

3-Methyl-5-(trifluoromethyl)-1*H*-pyrazol-4-yl]acetic acid used in the above procedure was prepared as follows:

(i) A solution of 4-bromo-3-methyl-5-(trifluoromethyl)-1*H*-pyrazole (11.45 g, 50 mmol) in tetrahydrofuran (250 ml) was stirred at 5˚C under argon. Sodium hydride (60% dispersion in oil, 2.0 g, 50 mmol) was added portionwise and the reaction mixture was stirred at 5˚C for 10 minutes. {2-[(Chloromethyl)oxy]ethyl}(trimethyl)silane chloride (8.33 g, 8.9 ml, 50 mmol) was added dropwise and the reaction was stirred at 5˚C for 90 minutes. The reaction was quenched by the careful addition of water and the mixture was extracted with ethyl acetate. The ethyl acetate extracts were combined, washed with water and brine, and then dried and evaporated. The residue was purified by column chromatography on silica gel eluting with ethyl acetate/hexanes (1:20 - 1:10) to give a mixture of 4-bromo-5-methyl-3-(trifluoromethyl)-1-({[2-(trimethylsilyl)ethyl]oxy}methyl)-1*H*-pyrazole and 4-bromo-3-methyl-5-(trifluoromethyl)-1-({[2-(trimethylsilyl)ethyl]oxy}methyl)-1*H*-pyrazole (13.55 g) which was used in the next step.

(ii) A solution of 4-bromo-5-methyl-3-(trifluoromethyl)-1-({[2-(trimethylsilyl)ethyl]oxy}methyl)-1*H*-pyrazole and 4-bromo-3-methyl-5-(trifluoromethyl)-1-({[2-(trimethylsilyl)ethyl]oxy}methyl)-1*H*-pyrazole (10 g, 28 mmol) in tetrahydrofuran (150 ml) was stirred at -78˚C under argon. n-Butyl lithium (11.2 ml, 2.5M solution in hexanes, 28 mmol) was added and the reaction was stirred at -78˚C for 30 minutes. *N,N*-Dimethylformamide (4.1 g, 4.3 ml, 56 mmol) was added dropwise and the reaction was stirred at -78˚C for 30 minutes and then warmed to room temperature and

stirred at room temperature for 1 hour. The reaction mixture was cooled to 0˚C and the reaction was quenched with citric acid solution. The solution was extracted with ethyl acetate, the organic layer was separated, washed with water and brine, and then dried and evaporated to give a mixture of 5-methyl-3-(trifluoromethyl)-1-({[2-(trimethylsilyl)ethyl]oxy}methyl)-1*H*-pyrazole-4-carbaldehyde and 3-methyl-5-(trifluoromethyl)-1-({[2-(trimethylsilyl)ethyl]oxy}methyl)-1*H*-pyrazole-4-carbaldehyde (8.34 g) which was used in the next step.

(iii) A solution of 5-methyl-3-(trifluoromethyl)-1-({[2-(trimethylsilyl)ethyl]oxy}methyl)-1*H*-pyrazole-4-carbaldehyde and 3-methyl-5-(trifluoromethyl)-1-({[2-(trimethylsilyl)ethyl]oxy}methyl)-1*H*-pyrazole-4-carbaldehyde (11.4 g, 37 mmol) in ethanol (100 ml) was stirred at room temperature. Sodium borohydride (0.700 g, 18.5 mmol) was added portionwise with ice-water cooling. The suspension was stirred at room temperature for 30 minutes and then cooled in an ice-water bath and quenched by the cautious addition of citric acid solution. Ethyl acetate was added and the mixture was stirred at room temperature for 5 minutes and the organic solvent was evaporated. The resulting solution was extracted with ethyl acetate and the organic layer was separated, washed with brine, dried and evaporated. The residue was purified by silica gel chromatography eluting with ethyl acetate/n-hexanes (1:4- 1:1) to give a mixture of [5-methyl-3-(trifluoromethyl)-1-({[2-(trimethylsilyl)ethyl]oxy}methyl)-1*H*-pyrazol-4-yl]methanol and [3-methyl-5-(trifluoromethyl)-1-({[2-(trimethylsilyl)ethyl]oxy}methyl)-1*H*-pyrazol-4-yl]methanol (7.7 g).

(iv) A solution of [5-methyl-3-(trifluoromethyl)-1-({[2-(trimethylsilyl)ethyl]oxy}methyl)-1*H*-pyrazol-4-yl]methanol and [3-methyl-5-(trifluoromethyl)-1-({[2-(trimethylsilyl)ethyl]oxy}methyl)-1*H*-pyrazol-4-yl]methanol (3.1 g, 10 mmol) in dichloromethane (50 ml) was stirred at 5˚C under argon. Triethylamine (2.02g, 2.8 ml, 20 mmol) was added followed by methanesulfonyl chloride (1.37 g, 0.9 ml, 12 mmol) and the reaction was stirred at 5˚C for 4 hours. The solution was washed with water and the organic layer was separated, then washed sequentially with citric acid, water and brine. The solution was dried over anhydrous magnesium sulphate, then filtered and evaporated to give a mixture of 4-(chloromethyl)-5-methyl-3-(trifluoromethyl)-1-({[2-(trimethylsilyl)ethyl]oxy}methyl)-1*H*-pyrazole and 4-(chloromethyl)-3-methyl-5-(trifluoromethyl)-1-({[2-(trimethylsilyl)ethyl]oxy}methyl)-1*H*-pyrazole (3.3 g) which was used in the next step.

(v) A solution of 4-(chloromethyl)-5-methyl-3-(trifluoromethyl)-1-({[2-(trimethylsilyl)ethyl]oxy}methyl)-1*H*-pyrazole and 4-(chloromethyl)-3-methyl-5-(trifluoromethyl)-1-({[2-(trimethylsilyl)ethyl]oxy}methyl)-1*H*-pyrazole (3.3 g, 10 mmol) in dimethylsulfoxide (20 ml) was treated with potassium cyanide( 0.650 g, 10 mmol) and the mixture was heated at 80˚C for 5 hours. The reaction was cooled to room temperature, diluted with water and the solution was extracted with chloroform. The organic extracts were combined, then washed with water and then with brine, dried and evaporated. The residue was purified by silica gel chromatography eluting with ethyl acetate/hexane (1:10 - 1: 2) to give a mixture of [5-methyl-3-(trifluoromethyl)-1-({[2-(trimethylsilyl)ethyl]oxy}methyl)-1*H*-pyrazol-4-yl]acetonitrile and [3-methyl-5-(trifluoromethyl)-1-({[2-(trimethylsilyl)ethyl]oxy}methyl)-1*H*-pyrazol-4-yl]acetonitrile (0.597 g) which was used in the next step.

(vi) A mixture of [5-methyl-3-(trifluoromethyl)-1-({[2-(trimethylsilyl)ethyl]oxy}methyl)-1*H*-pyrazol-4-yl]acetonitrile and [3-methyl-5-(trifluoromethyl)-1-({[2-(trimethylsilyl)ethyl]oxy}methyl)-1*H*-pyrazol-4-yl]acetonitrile (0.597 g, 1.9 mmol) in dioxane (4 ml) and hydrochloric acid (5N, 4ml) was heated at reflux for 24 hours. Additional hydrochloric acid (5N, 2 ml) was added and the reaction was heated at reflux for a further 6 hours. After cooling to room temperature the solvent was evaporated and the residue was co-evaporated with ,toluene and ether. The residue was dried to give crude 3-methyl-5-(trifluoromethyl)-1*H*-pyrazol-4-yl]acetic acid which was used in subsequent reactions without further purification.

Examples 35-36

In a manner analogous to that described for Example 34 above the compounds tabulated below (Table 3) were prepared by substituting the appropriate amine (or salt thereof) for the [(2-chloro-4-fluorophenyl)methyl]amine used in the above procedure. All of the amines required to prepare the examples listed in Table 3 are available from commercial sources or can be prepared using routes described previously in the chemical literature unless stated otherwise,

Table 3

| Example no. | Chemical name | [M+H]$^+$ | Retention time (mins) |
|---|---|---|---|
| E35 | 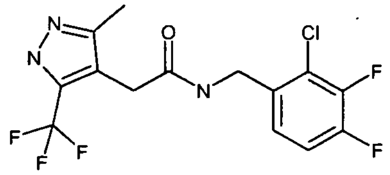 *N*-[(2-Chloro-3,4- difluorophenyl)methyl]-2-[3- methyl-5-(trifluoromethyl)-1*H*-pyrazol-4-yl]acetamide | 368 | 2.52 |
| E36 | 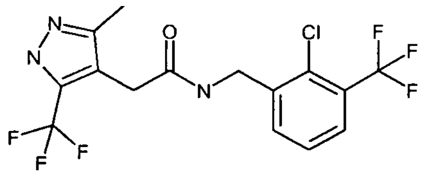 *N*-{[2-Chloro-3-(trifluoromethyl)phenyl]methyl}-2-[3-methyl-5-(trifluoromethyl)-1*H*-pyrazol-4-yl]acetamide | 400 | 2.71 |

Example 37 2-(3,5-Dimethyl-1*H*-pyrazol-4-yl)-*N*-[1,2,3,4-tetrahydro-1-naphthalenyl]acetamide (E37)

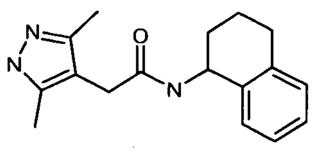

(3,5-Dimethyl-1*H*-pyrazol-4-yl)acetic acid (0.100 g, 0.35 mmol) was dissolved in dichloromethane (10 ml) and to this was added water soluble carbodiimide (0.162 g, 0.84 mmol), 1-hydroxybenzotriazole (0.114 g, 0.84 mmol), N-ethyl morpholine (0.413 ml, 3.25 mmol), and (1*S*)-1,2,3,4-tetrahydro-1-naphthalenylamine (0.124 g, 0.52 mmol). The mixture was stirred overnight at room temperature and then diluted with more dichloromethane (10 ml) and washed with saturated aqueous sodium hydrogen carbonate (20 ml). The organic phase was separated by filtration through a hydrophobic frit and evaporated to give the crude product as a yellow solid. The crude material was purified by mass-directed automated HPLC to give pure 2-(3,5-dimethyl-1*H*-pyrazol-4-yl)-*N*-[1,2,3,4-tetrahydro-1-naphthalenyl]acetamide as a white solid after freeze-drying of the collected product fractions (0.029 g).
LC/MS [M+H]$^+$ = 284 retention time = 2.00 minutes.

Mass-directed automated HPLC

Purification by mass-directed automated HPLC was carried out using the following apparatus and conditions:

Hardware

Waters 2525 Binary Gradient Module
Waters 515 Makeup Pump
Waters Pump Control Module
Waters 2767 Inject Collect
Waters Column Fluidics Manager
Waters 2996 Photodiode Array Detector
Waters ZQ Mass Spectrometer
Gilson 202 fraction collector

Gilson Aspec waste collector

Software

Waters MassLynx version 4 SP2

Column

The columns used are Waters Atlantis, the dimensions of which are 19mm x 100mm (small scale) and 30mm x 100mm (large scale). The stationary phase particle size is 5μm.

Solvents

A : Aqueous solvent = Water + 0.1% Formic Acid
B : Organic solvent = Acetonitrile + 0.1 % Formic Acid
Make up solvent = Methanol : Water 80:20
Needle rinse solvent = Methanol

Methods

There are five methods used depending on the analytical retention time of the compound of interest. They have a 13.5-minute runtime, which comprises a 10-minute gradient followed by a 3.5 minute column flush and re-equilibration step.

Large/Small Scale 1.0-1.5 = 5-30% B

Large/Small Scale 1.5-2.2 = 15-55% B

Large/Small Scale 2.2-2.9 = 30-85% B

Large/Small Scale 2.9-3.6 = 50-99% B

Large/Small Scale 3.6-5.0 = 80-99% B

(in 6 minutes followed by 7.5 minutes flush and re-equilibration)

Flow rate

All of the above methods have a flow rate of either 20mls/min (Small Scale) or 40mls/min (Large Scale).

Liquid Chromatography / Mass Spectrometry

Analysis of the above Examples by Liquid Chromatography / Mass Spectrometry (LC/MS) was carried out using the following apparatus and conditions:

Hardware

Agilent 1100 Gradient Pump
Agilent 1100 Autosampler
Agilent 1100 DAD Detector
Agilent 1100 Degasser
Agilent 1100 Oven
Agilent 1100 Controller
Waters ZQ Mass Spectrometer
Sedere Sedex 85

Software

Waters MassLynx version 4.0 SP2

Column

The column used is a Waters Atlantis, the dimensions of which are 4.6mm x 50mm. The stationary phase particle size is 3μm.

Solvents

A : Aqueous solvent = Water + 0.05% Formic Acid
B : Organic solvent = Acetonitrile + 0.05% Formic Acid

Method

The generic method used has a 5 minute runtime.

| Time / min | %B |
|---|---|
| 0 | 3 |
| 0.1 | 3 |
| 4 | 97 |
| 4.8 | 97 |
| 4.9 | 3 |
| 5.0 | 3 |

The above method has a flow rate of 3ml/mins.
The injection volume for the generic method is 5ul.
The column temperature is 30deg.
The UV detection range is from 220 to 330nm.

Pharmacological data

Compounds of the invention may be tested for *in vitro* biological activity at the P2X7 receptor in accordance with the following studies:

Ethidium Accumulation Assay

Studies were performed using NaCl assay buffer of the following composition (in mM): 140 NaCl, HEPES 10, *N*-methyl-D-glucamine 5, KCl 5.6, D-glucose 10, $CaCl_2$ 0.5 (pH 7.4). HEK293 cells, expressing human recombinant P2X7 receptors, were grown in poly-L-lysine pretreated 96 well plates for 18-24 h. (The cloning of the human P2X7 receptor is described in US 6,133,434). The cells were washed twice with 350μl of assay buffer before addition of 50μl of test compound. The cells were then incubated at room temperature (19-21°C) for 30 min before addition of ATP and ethidium (100μM final assay concentration). The ATP concentration was chosen to be close to the $EC_{80}$ for the receptor type and was 1 mM for studies on the human P2X7 receptor. Incubations were continued for 8 or 16 min and were terminated by addition of 25μl of 1.3M sucrose containing 5mM of the P2X7 receptor antagonist reactive black 5 (Aldrich). Cellular accumulation of ethidium was determined by measuring fluorescence (excitation wavelength of 530nm and emission wavelength of 620nm) from below the plate with a Canberra Packard Fluorocount (Pangbourne, UK) or a Flexstation.II (Molecular Devices). Antagonist $pIC_{50}$ values for blocking ATP responses were determined using iterative curve fitting techniques.

Fluorescent Imaging Plate Reader (FLIPR) Ca Assay

Studies were performed using NaCl assay buffer of the following composition (in mM) for human P2X7: NaCl 137; HEPES 20; KCl; 5.37; $NaHCO_3$ 4.17; $CaCl_2$ 1; $MgSO_4$ 0.5; and 1g/L of D-glucose (pH 7.4). HEK293 cells, expressing human recombinant P2X7 receptors, were grown in poly-L-lysine pretreated 384 well plates for 42-48h. (The cloning of the human P2X7 receptor is described in US 6,133,434). The cells were washed three times with 80μl of assay buffer, loaded for 1 h at 37°C with 2μM Fluo4 (Teflabs), washed three times again, and left with 30μl buffer before the addition of 10 μl of 4x concentrated test compound. The cells were then incubated at room temperature for 30 mins before addition (online, by FLIPR384 or FLIPR3 instrument (Molecular Devices)) of Benzoylbenzoyl-ATP (BzATP) 60μM final assay concentration. The BzATP concentration was chosen to be close to the $EC_{80}$ for the receptor type. Incubations and reading were continued for 90sec, and intracellular calcium increase was determined by measuring fluorescence (excitation wavelength of 488nm and emission wavelength of 516nm) from below the plate, with FLIPR CCD camera. Antagonist $pIC_{50}$ values for blocking BzATP responses were determined using iterative curve fitting techniques.

The compounds of Examples 1-37 were tested in the FLIPR Ca Assay and/or the Ethidium Accumulation Assay for human P2X7 receptor antagonist activity and found to have pIC50 values > 4.7 in the FLIPR Ca Assay and/or pIC50 values > 5.5 in the Ethidium Accumulation Assay.

**Claims**

1. A compound of formula (I), or a pharmaceutically acceptable salt thereof:

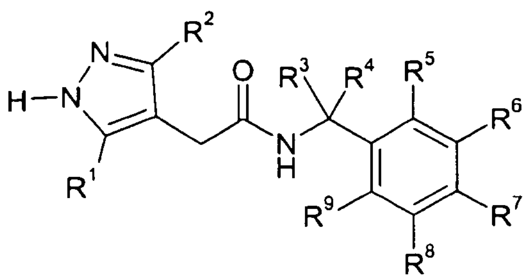

(I)

wherein:

$R^1$ and $R^2$ represent $C_{1-6}$ alkyl, phenyl, or a $C_{3-6}$ cycloalkyl, any of which is optionally substituted with 1, 2 or 3 halogen atoms;
$R^3$ and $R^4$ independently represent hydrogen or $C_{1-3}$ alkyl;
$R^5$, $R^6$, $R^7$, $R^8$ and $R^9$ independently represent hydrogen, halogen, cyano, $C_{1-6}$ alkyl, $C_{2-6}$ alkenyl, $C_{2-6}$ alkynyl, $C_{3-6}$ cycloalkyl or phenyl, and any of said $C_{1-6}$ alkyl, $C_{2-6}$ alkenyl, $C_{2-6}$ alkynyl, $C_{3-6}$ cycloalkyl or phenyl is optionally substituted with 1, 2 or 3 halogen atoms; or $R^8$ and $R^9$ together with the carbon

atoms to which they are attached form a benzene ring which is optionally substituted with 1, 2 or 3 halogen atoms; or $R^4$ and $R^5$ together with the carbon atoms to which they are attached form a $C_{5-7}$ cycloalkyl;
with the proviso that when $R^5$ and $R^9$ are both selected from hydrogen or fluorine, at least one of $R^6$, $R^7$ and $R^8$ is a halogen atom, or $R^6$, $R^7$ and $R^8$ are selected from the group consisting of hydrogen, methyl and $CF_3$ and one, but not more than one, of $R^6$,
$R^7$ and $R^8$ is methyl or $CF_3$.

**2.** A compound of formula (I), or a pharmaceutically acceptable salt thereof, as defined in claim 1, wherein $R^1$ and $R^2$ independently represent unsubstituted $C_{1-6}$ alkyl, trifluoromethyl, phenyl or a $C_{3-6}$ cycloalkyl.

**3.** A compound of formula (I), or a pharmaceutically acceptable salt thereof, as defined in claim 1 or 2, wherein $R^3$ and $R^4$ both represent hydrogen.

**4.** A compound of formula (I), or a pharmaceutically acceptable salt thereof, as defined in any one of claims 1 to 3, wherein $R^5$, $R^6$, $R^7$, $R^8$ and $R^9$ independently represent hydrogen, halogen, cyano, trifluoromethyl or methyl.

**5.** A compound of formula (I) as defined in claim 1 which is:

*N*-[(2-Chloro-4-fluorophenyl)methyl]-2-[3-methyl-5-(2-methylpropyl)-1*H*-pyrazol-4-yl]acetamide (E1)

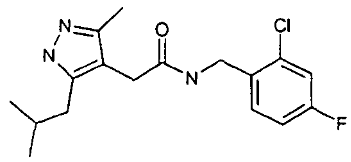

;

*N*-[(2-Chloro-4-fluorophenyl)methyl]-2-(3-methyl-5-phenyl-1*H*-pyrazol-4-yl)acetamide (E2)

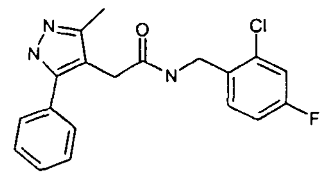

;

*N*-[(2-Chloro-4-fluorophenyl)methyl]-2-[5-(1,1-dimethylethyl)-3-methyl-1*H*-pyrazol-4-yl]acetamide (E3)

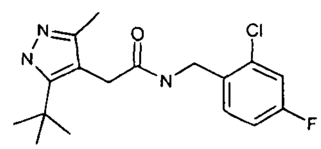

;

*N*-[(2-Chloro-4-fluorophenyl)methyl]-2-(3,5-dimethyl-1*H*-pyrazol-4-yl)acetamide (E4)

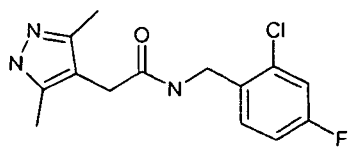

;

2-(3,5-Dimethyl-1*H*-pyrazol-4-yl)-*N*-[(2,3,4-trifluorophenyl)methyl]acetamide (E5)

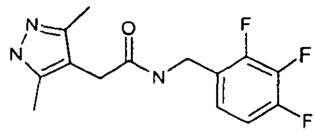

;

N-[(2-Bromophenyl)methyl]-2-(3,5-dimethyl-1H-pyrazol-4-yl)acetamide (E6)

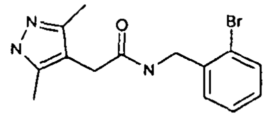

;

N-[(4-Chlorophenyl)methyl]-2-(3,5-dimethyl-1H-pyrazol-4-yl)acetamide (E7)

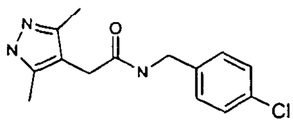

;

N-[(2,4-Dichlorophenyl)methyl]-2-(3,5-dimethyl-1H-pyrazol-4-yl)acetamide (E8)

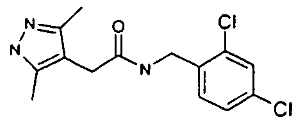

;

N-[(3,4-Dichlorophenyl)methyl]-2-(3,5-dimethyl-1H-pyrazol-4-yl)acetamide (E9)

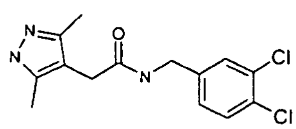

;

N-[(3,5-Dichlorophenyl)methyl]-2-(3,5-dimethyl-1H-pyrazol-4-yl)acetamide (E10)

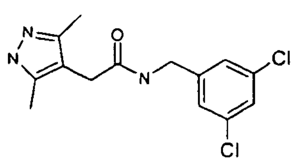

;

N-[(2,6-Dichlorophenyl)methyl]-2-(3,5-dimethyl-1H-pyrazol-4-yl)acetamide (E11)

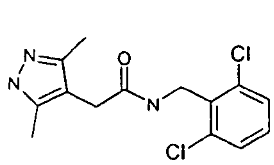

;

*N*-[(2-Chloro-6-methylphenyl)methyl]-2-(3,5-dimethyl-1*H*-pyrazol-4-yl)acetamide (E12)

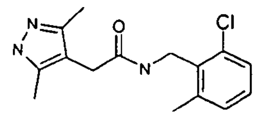

;

*N*-[(3-Chlorophenyl)methyl]-2-(3,5-dimethyl-1*H*-pyrazol-4-yl)acetamide (E13)

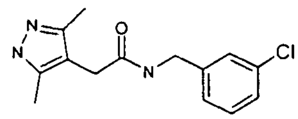

;

*N*-[(2-Chloro-5-fluorophenyl)methyl]-2-(3,5-dimethyl-1*H*-pyrazol-4-yl)acetamide (E14)

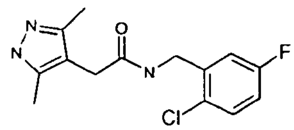

;

*N*-[(3-Chloro-2-methylphenyl)methyl]-2-(3,5-dimethyl-1*H*-pyrazol-4-yl)acetamide (E15)

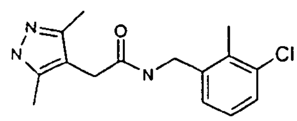

;

*N*-[(2-Chloro-6-fluorophenyl)methyl]-2-(3,5-dimethyl-1*H*-pyrazol-4-yl)acetamide (E16)

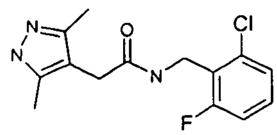

;

*N*-[(2,5-Dichlorophenyl)methyl]-2-(3,5-dimethyl-1*H*-pyrazol-4-yl)acetamide (E17)

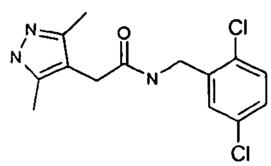

;

*N*-[(2-Chlorophenyl)methyl]-2-(3,5-dimethyl-1*H*-pyrazol-4-yl)acetamide (E18)

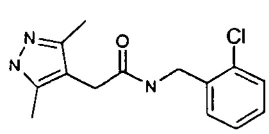

;

*N*-[(2,4-Difluorophenyl)methyl]-2-(3,5-dimethyl-1*H*-pyrazol-4-yl)acetamide (E19)

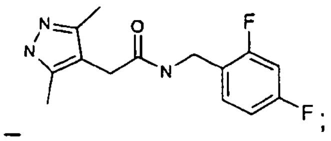

2-(3,5-Dimethyl-1*H*-pyrazol-4-yl)-*N*-{[4-fluoro-3-(trifluoromethyl)phenyl]methyl}acetamide (E20)

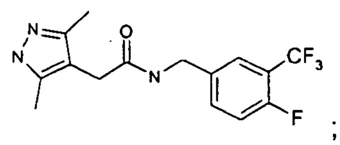

2-(3,5-Dimethyl-1*H*-pyrazol-4-yl)-*N*-{[4-fluoro-2-(trifluoromethyl)phenyl]methyl}acetamide (E21)

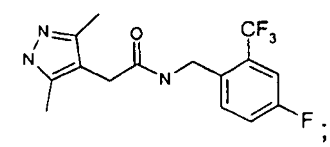

*N*-{[4-Chloro-3-(trifluoromethyl)phenyl]methyl}-2-(3,5-dimethyl-1*H*-pyrazol-4-yl)acetamide (E22)

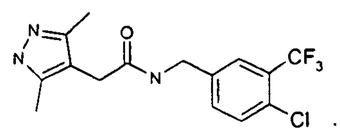

*N*-[(2,4-Dimethylphenyl)methyl]-2-(3,5-dimethyl-1*H*-pyrazol-4-yl)acetamide (E23)

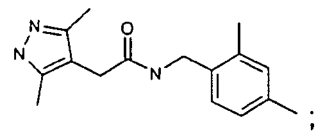

*N*-{[2-Chloro-3-(trifluoromethyl)phenyl]methyl}-2-(3,5-dimethyl-1*H*-pyrazol-4-yl)acetamide (E24)

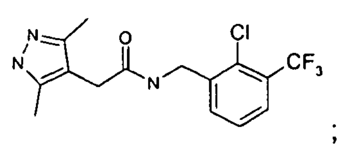

*N*-[(2-Chloro-6-fluoro-3-methylphenyl)methyl]-2-(3,5-dimethyl-1*H*-pyrazol-4-yl)acetamide (E25)

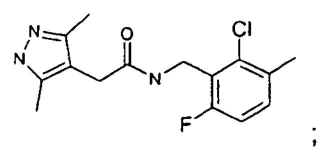

*N*-[(4-Bromo-2-fluorophenyl)methyl]-2-(3,5-dimethyl-1*H*-pyrazol-4-yl)acetamide (E26)

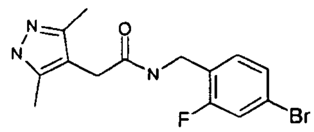

;

*N*-[(6-Chloro-2-fluoro-3-methylphenyl)methyl]-2-(3,5-dimethyl-1*H*-pyrazol-4-yl)acetamide (E27)

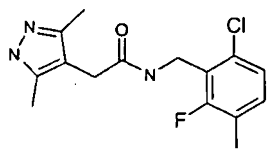

;

2-(3,5-Dimethyl-1*H*-pyrazol-4-yl)-*N*-[(2-methylphenyl)methyl]acetamide (E28)

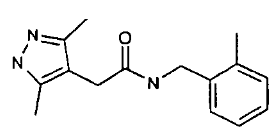

;

2-(3,5-Dimethyl-1*H*-pyrazol-4-yl)-*N*-{[4-(trifluoromethyl)phenyl]methyl}acetamide (E29)

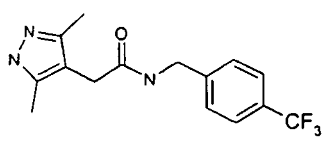

;

*N*-[(2-Chloro-4-fluorophenyl)methyl]-2-(3,5-diethyl-1*H*-pyrazol-4-yl)acetamide (E30)

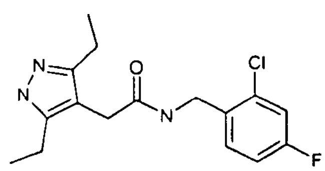

;

2-(3,5-Diethyl-1*H*-pyrazol-4-yl)-*N*-[(2,3,4-trifluorophenyl)methyl]acetamide (E31)

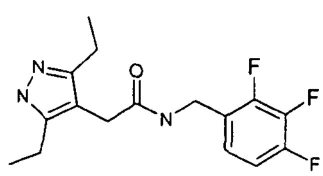

;

2-[3,5-Bis(1-methylethyl)-1*H*-pyrazol-4-yl]-*N*-[(2-chloro-4-fluorophenyl)methyl]acetamide (E32)

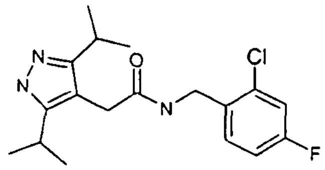

;

2-[3,5-Bis(1-methylethyl)-1*H*-pyrazol-4-yl]-*N*-[(2,3,4-trifluorophenyl)methyl]acetamide (E33)

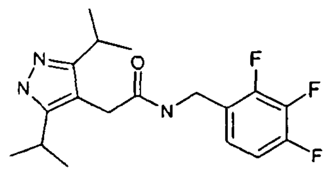

;

*N*-[(2-Chloro-4-fluorophenyl)methyl]-2-[3-methyl-5-(trifluoromethyl)-1*H*-pyrazol-4-yl]acetamide (E34)

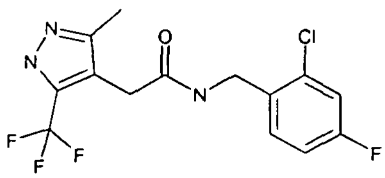

;

*N*-[(2-Chloro-3,4-difluorophenyl)methyl]-2-[3-methyl-5-(trifluoromethyl)-1*H*-pyrazol-4-yl]acetamide (E35)

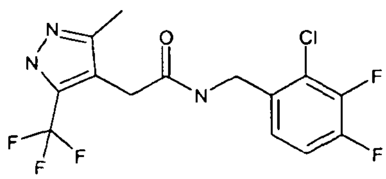

;

*N*-{[2-Chloro-3-(trifluoromethyl) phenyl] methyl}-2-[3-methyl-5-(trifluoromethyl)-1*H*-pyrazol-4-yl] acetamide (E36)

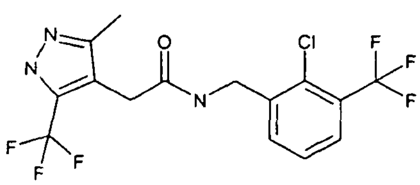

;

2-(3,5-Dimethyl-1*H*-pyrazol-4-yl)-*N*-[1,2,3,4-tetrahydro-1-naphthalenyl]acetamide (E37)

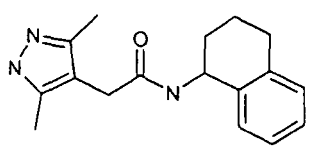

,

or a pharmaceutically acceptable salt thereof.

**6.** A pharmaceutical composition which comprises a compound of formula (I) or a pharmaceutically acceptable salt thereof as defined in any preceding claim and a pharmaceutically acceptable carrier or excipient.

**7.** A compound, or pharmaceutically acceptable salt thereof, as defined in any one of claims 1 to 5 for use in therapy.

**8.** A compound of formula (I) as claimed in any one of claims 1 to 5 or a pharmaceutically acceptable salt thereof for use in the treatment of a human or animal subject suffering from pain, inflammation or a neurodegenerative disease.

**9.** Use of a compound, or a pharmaceutically acceptable salt thereof, as defined in any one of claims 1 to 5 for the manufacture of a medicament for the treatment of pain, inflammation or a neurodegenerative disease.

## Patentansprüche

**1.** Eine Verbindung der Formel (I) oder ein pharmazeutisch verträgliches Salz davon,

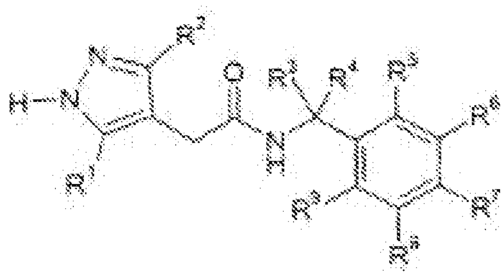

(I)

wobei:

$R^1$ und $R^2$ $C_{1-6}$-Alkyl, Phenyl oder ein $C_{3-6}$-Cycloalkyl bedeuten, von denen jedes gegebenenfalls mit 1, 2 oder 3 Halogenatomen substituiert ist;
$R^3$ und $R^4$ unabhängig Wasserstoff oder $C_{1-3}$-Alkyl bedeuten;
$R^5$, $R^6$, $R^7$, $R^8$ und $R^9$ unabhängig Wasserstoff, Halogen, Cyano, $C_{1-6}$-Alkyl, $C_{2-6}$-Alkenyl, $C_{2-6}$-Alkinyl, $C_{3-6}$-Cycloalkyl oder Phenyl bedeuten, und jedes von $C_{1-6}$-Alkyl, $C_{2-6}$-Alkenyl, $C_{2-6}$-Alkinyl, $C_{3-6}$-Cycloalkyl oder Phenyl gegebenenfalls mit 1, 2 oder 3 Halogenatomen substituiert ist; oder $R^8$ und $R^9$ zusammen mit den Kohlenstoffatomen, an die sie gebunden sind, einen Benzolring bilden, der gegebenenfalls mit 1, 2 oder 3 Halogenatomen substituiert ist;
oder $R^4$ und $R^5$ zusammen mit den Kohlenstoffatomen, an die sie gebunden sind, ein $C_{5-7}$-Cycloalkyl bilden;
mit der Maßgabe, dass wenn $R^5$ und $R^9$ beide aus Wasserstoff oder Fluor ausgewählt sind, mindestens einer von $R^6$, $R^7$ und $R^8$ ein Halogenatom ist oder $R^6$, $R^7$ und $R^8$ aus der Gruppe bestehend aus Wasserstoff, Methyl und $CF_3$ ausgewählt sind und einer, aber nicht mehr als einer von $R^6$, $R^7$ und $R^8$ Methyl oder $CF_3$ ist.

**2.** Eine Verbindung der Formel (I) oder ein pharmazeutisch verträgliches Salz davon wie in Anspruch 1 definiert, wobei $R^1$ und $R^2$ unabhängig unsubstituiertes $C_{1-6}$-Alkyl, Trifluormethyl, Phenyl oder ein $C_{3-6}$-Cycloalkyl bedeuten.

**3.** Eine Verbindung der Formel (I) oder ein pharmazeutisch verträgliches Salz davon wie in Anspruch 1 oder 2 definiert, wobei $R^3$ und $R^4$ beide Wasserstoff bedeuten.

**4.** Eine Verbindung der Formel (I) oder ein pharmazeutisch verträgliches Salz davon wie in einem der Ansprüche 1 bis 3 definiert, wobei $R^5$, $R^6$, $R^7$, $R^8$ und $R^9$ unabhängig Wasserstoff, Halogen, Cyano, Trifluormethyl oder Methyl bedeuten.

**5.** Eine Verbindung der Formel (I) wie in Anspruch 1 definiert, nämlich:

N-[(2-Chlor-4-fluorphenyl)methyl]-2-[3-methyl-5-(2-methylpropyl)-1H-pyrazol-4-yl]acetamid (E1)

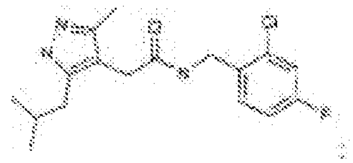

N-[(2-Chlor-4-fluorphenyl)methyl]-2-(3-methyl-5-phenyl-1H-pyrazol-4-yl)acetamid (E2)

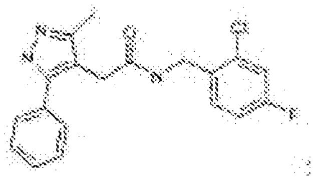

N-[(2-Chlor-4-fluorphenyl)methyl]-2-[5-(1,1-dimethylethyl)-3-methyl-1H-pyrazol-4-yl]acetamid (E3)

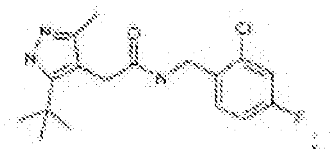

N-[(2-Chlor-4-fluorphenyl)methyl]-2-(3,5-dimethyl-1H-pyrazol-4-yl)acetamid (E4)

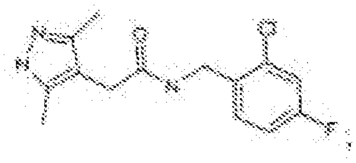

le 2-(3,5-Dimethyl-1H-pyrazol-4-yl)-N-[(2,3,4-trifluoro-phenyl)methyl]acetamide (E5)

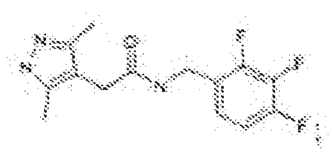

N-[(2-Bromophenyl)methyl]-2-(3,5-dimethyl-1H-pyrazol-4-yl)acetamid (E6)

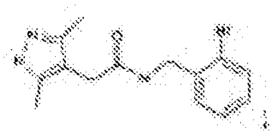

N-[(4-Chlorphenyl)methyl]-2-(3,5-dimethyl-1H-pyrazol-4-yl)acetamid (E7)

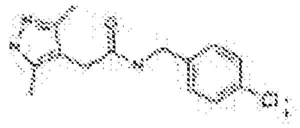

N-[(2,4-Dichlorphenyl)methyl]-2-(3,5-dimethyl-1H-pyrazol-4-yl)acetamid (E8)

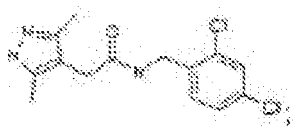

N-[(3,4-Dichlorphenyl)methyl]-2-(3,5-dimethyl-1H-pyrazol-4-yl)acetamid (E9)

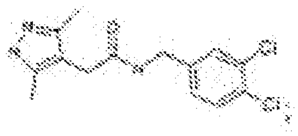

N-[(3,5-Dichlorphenyl)methyl]-2-(3,5-dimethyl-1H-pyrazol-4-yl)acetamid (E10)

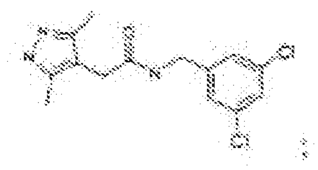

N-[(2,6-Dichlorphenyl)methyl]-2-(3,5-dimethyl-1H-pyrazol-4-yl)acetamid (E11)

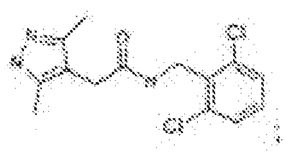

N-[(2-Chlor-6-methylphenyl)methyl]-2-(3,5-dimethyl-1H-pyrazol-4-yl)acetamid (E12)

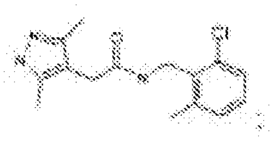

N-[(3-Chlorphenyl)methyl]-2-(3,5-dimethyl-1H-pyrazol-4-yl)acetamid (E13)

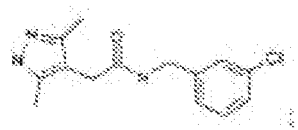

N-[(2-Chlor-5-fluorphenyl)methyl]-2-(3,5-dimethyl-1H-pyrazol-4-yl)acetamid (E14)

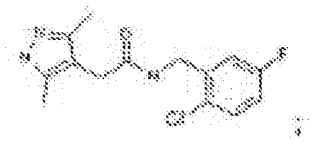

N-[(3-Chlor-2-methylphenyl)methyl]-2-(3,5-dimethyl-1H-pyrazol-4-yl)acetamid (E15)

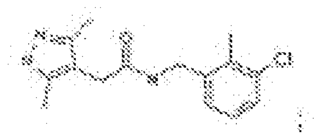

N-[(2-Chlor-6-fluorphenyl)methyl]-2-(3,5-dimethyl-1H-pyrazol-4-yl)acetamid (E16)

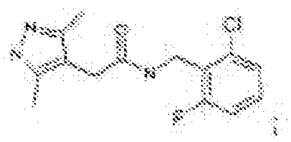

N-[(2,5-Dichlorphenyl)methyl]-2-(3,5-dimethyl-1H-pyrazol-4-yl)acetamid (E17)

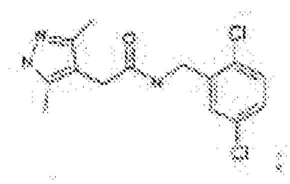

N-[(2-Chlorphenyl)methyl]-2-(3,5-dimethyl-1H-pyrazol-4-yl)acetamid (E18)

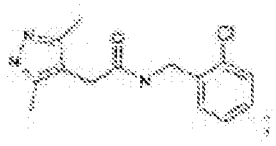

N-[(2,4-Difluorphenyl)methyl]-2-(3,5-dimethyl-1H-pyrazol-4-yl)acetamid (E19)

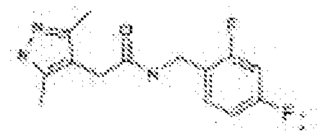

2-(3,5-Dimethyl-1H-pyrazol-4-yl)-N-{[4-fluor-3-(trifluormethyl)phenyl]methyl}acetamid

(E20)

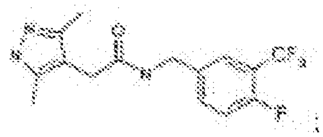

2-(3,5-Dimethyl-1H-pyrazol-4-yl)-N-{[4-fluor-2-(trifluormethyl)phenyl]methyl}acetamid (E21)

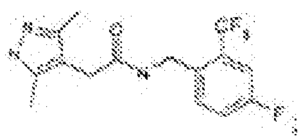

N-{[4-Chlor-3-(trifluormethyl)phenyl]methyl}-2-(3,5-dimethyl-1H-pyrazol-4-yl)acetamid (E22)

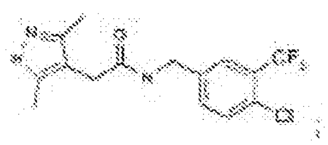

N-[(2,4-Dimethylphenyl)methyl]-2-(3,5-dimethyl-1H-pyrazol-4-yl)acetamid (E23)

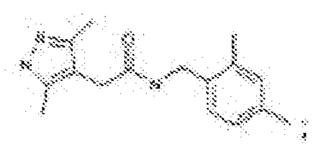

N-{[2-Chlor-3-(trifluormethyl)phenyl]methyl}-2-(3,5-dimethyl-1H-pyrazol-4-yl)acetamid (E24)

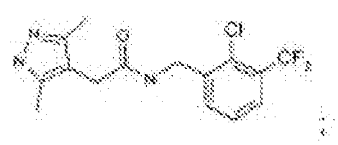

N-[(2-Chlor-6-fluor-3-methylphenyl)methyl]-2-(3,5-dimethyl-1H-pyrazol-4-yl)acetamid (E25)

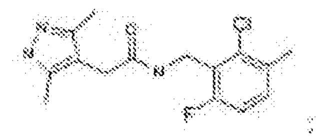

N-[(4-Brom-2-fluorphenyl)methyl]-2-(3,5-dimethyl-1H-pyrazol-4-yl)acetamid (E26)

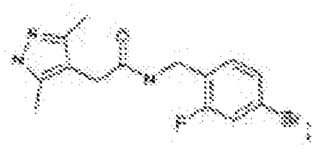

N-[(6-Chlor-2-fluor-3-methylphenyl)methyl]-2-(3,5-dimethyl-1H-pyrazol-4-yl)acetamid (E27)

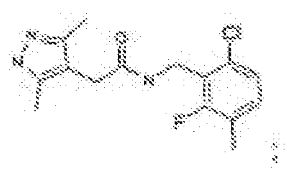

2-(3,5-Dimethyl-1H-pyrazol-4-yl)-N-[(2-methylphenyl)methyl]acetamid (E28)

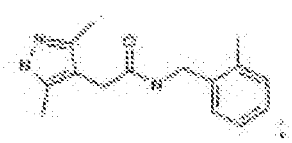

2-(3,5-Dimethyl-1H-pyrazol-4-yl)-N-{[4-(trifluormethyl)phenyl]methyl}acetamid (E29)

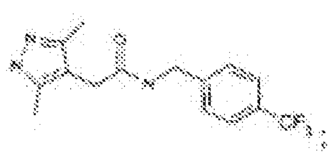

N-[(2-Chlor-4-fluorphenyl)methyl]-2-(3,5-diethyl-1H-pyrazol-4-yl)acetamid (E30)

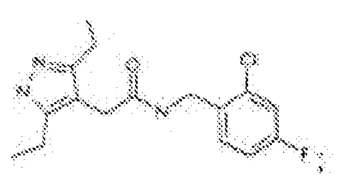

2-(3,5-Diethyl-1H-pyrazol-4-yl)-N-[(2,3,4-trifluorphenyl)methyl]acetamid (E31)

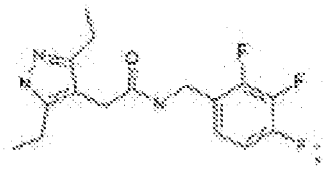

2-[3,5-Bis(1-methylethyl)-1H-pyrazol-4-yl]-N-[(2-chlor-4-fluorphenyl)methyl]acetamid (E32)

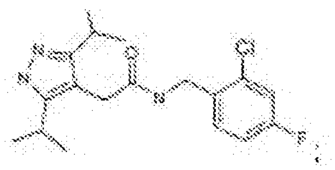

2-[3,5-Bis(1-methylethyl)-1H-pyrazol-4-yl]-N-[(2,3,4-trifluorphenyl)methyl]acetamid (E33)

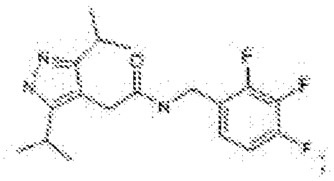

N-[(2-Chlor-4-fluorphenyl)methyl]-2-[3-methyl-5-(trifluormethyl)-1H-pyrazol-4-yl]acetamid (E34)

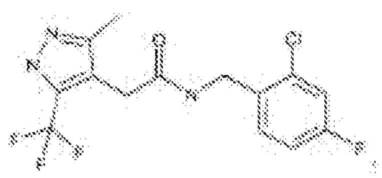

N-[(2-Chlor-3,4-difluorphenyl)methyl]-2-[3-methyl-5-(trifluormethyl)-1H-pyrazol-4-yl]acetamid (E35)

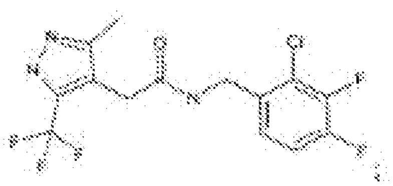

N-{[2-Chlor-3-(trifluormethyl)phenyl]methyl}-2-[3-methyl-5-(trifluormethyl)-1H-pyrazol-4-yl]acetamid (E36)

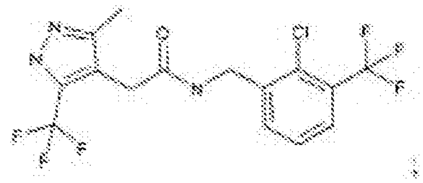

2-(3,5-Dimethyl-1H-pyrazol-4-yl)-N-[1,2,3,4-tetrahydro-1-naphthalenyl]acetamid (E37)

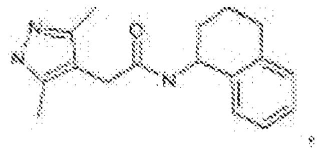

oder ein pharmazeutisch verträgliches Salz davon.

**6.** Ein Arzneimittel, welches eine Verbindung der Formel (I) oder ein pharmazeutisch verträgliches Salz davon wie in einem vorhergehenden Anspruch definiert und einen pharmazeutisch verträglichen Träger oder Exzipienten umfasst.

**7.** Eine Verbindung oder ein pharmazeutisch verträgliches Salz davon wie in einem der Ansprüche 1 bis 5 definiert zur Verwendung bei der Therapie.

**8.** Eine Verbindung der Formel (I) wie in einem der Ansprüche 1 bis 5 beansprucht oder ein pharmazeutisch verträgliches Salz davon zur Verwendung bei der Behandlung eines menschlichen oder tierischen Patienten, der unter Schmerz, Entzündung oder einer neurodegenerativen Erkrankung leidet.

**9.** Verwendung einer Verbindung oder eines pharmazeutisch verträglichen Salzes davon wie in einem der Ansprüche 1 bis 5 definiert zur Herstellung eines Medikaments zur Behandlung von Schmerz, Entzündung oder einer neurodegenerativen Erkrankung.

**Revendications**

**1.** Composé de formule (I), ou un de ses sels pharmaceutiquement acceptables :

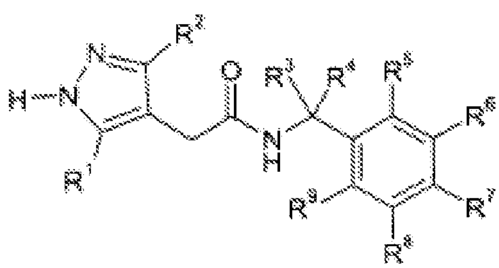

(I)

formule dans laquelle :

$R^1$ et $R^2$ représentent un groupe alkyle en $C_1$ à $C_6$, phényle ou cycloalkyle en $C_3$ à $C_6$, n'importe lequel de ces groupes étant facultativement substitué avec 1, 2 ou 3 atomes d'halogènes ;
$R^3$ et $R^4$ représentent indépendamment un atome d'hydrogène ou un groupe alkyle en $C_1$ à $C_3$ ;
$R^5$, $R^6$, $R^7$, $R^8$ et $R^9$ représentent indépendamment un atome d'hydrogène, un atome d'halogène, un groupe cyano, alkyle en $C_1$ à $C_6$, alcényle en $C_2$ à $C_6$, alcynyle en $C_2$ à $C_6$, cycloalkyle en $C_3$ à $C_6$ ou phényle, et n'importe lequel desdits groupes alkyle en $C_1$ à $C_6$, alcényle en $C_2$ à $C_6$, alcynyle en $C_2$ à $C_6$, cycloalkyle en $C_3$ à $C_6$ et phényle est facultativement substitué avec 1, 2 ou 3 atomes d'halogènes ; ou bien $R^8$ et $R^9$, conjointement avec les atomes de carbone auxquels ils sont fixés, forment un noyau benzénique qui est facultativement substitué avec 1, 2 ou 3 atomes d'halogènes ;
ou bien $R^4$ et $R^5$, conjointement avec les atomes de carbone auxquels ils sont fixés, forment un groupe cycloalkyle en $C_5$ à $C_7$ ;
sous réserve que, lorsque $R^5$ et $R^9$ sont l'un et l'autre choisis entre un atome d'hydrogène et un atome de fluor, au moins un de $R^6$, $R^7$ et $R^8$ représente un atome d'halogène, ou bien $R^6$, $R^7$ et $R^8$ sont choisis dans le groupe

consistant en un atome d'hydrogène, un groupe méthyle et un groupe $CF_3$ et un, mais pas plus d'un, de $R^6$, $R^7$ et $R^8$ représente un groupe méthyle ou un groupe $CF_3$.

**2.** Composé de formule (I), ou un de ses sels pharmaceutiquement acceptables, suivant la revendication 1, dans lequel $R^1$ et $R^2$ représentent indépendamment un groupe alkyle en $C_1$ à $C_6$ non substitué, trifluorométhyle, phényle ou cycloalkyle en $C_3$ à $C_6$.

**3.** Composé de formule (I), ou un de ses sels pharmaceutiquement acceptables, suivant la revendication 1 ou 2, dans lequel $R^3$ et $R^4$ représentent l'un et l'autre un atome d'hydrogène.

**4.** Composé de formule (I), ou un de ses sels pharmaceutiquement acceptables, suivant l'une quelconque des revendications 1 à 3, dans lequel $R^5$, $R^6$, $R^7$, $R^8$ et $R^9$ représentent indépendamment un atome d'hydrogène, un atome d'halogène, un groupe cyano, trifluorométhyle ou méthyle.

**5.** Composé de formule (I) suivant la revendication 1, qui est :

le *N*-[(2-chloro-4-trifluorophényl)méthyl]-2-[3-méthyl-5-(2-méthylpropyl)-1*H*-pyrazole-4-yl]acétamide (E1)

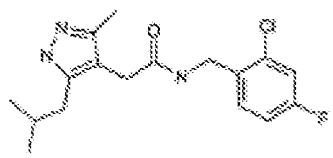

;

le *N*-[(2-chloro-4-fluorophényl)méthyl]-2-(3-méthyl-5-phényl-1*H*-pyrazole-4-yl)acétamide (E2)

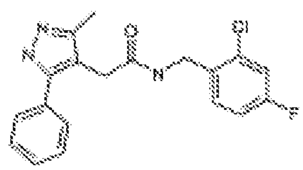

;

le *N*-[(2-chloro-4-fluorophényl)méthyl]-2-[5-(1,1-diméthyl-éthyl)-3-méthyl-1*H*-pyrazole-4-yl]acétamide (E3)

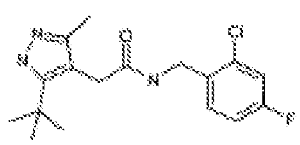

;

le *N*-[(2-chloro-4-fluorophényl)méthyl]-2-(3,5-diméthyl-1*H*-pyrazole-4-yl)acétamide (E4)

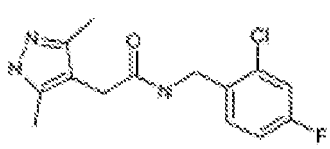

;

le 2-(3,5-diméthyl-1*H*-pyrazole-4-yl)-*N*-[(2,3,4-trifluorophényl)méthyl]acétamide (E5)

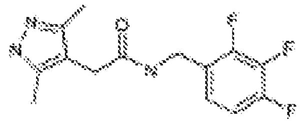
;

le *N*-[(2-bromophényl)méthyl]-2-(3,5-diméthyl-1*H*-pyrazole-4-yl)acétamide (E6)

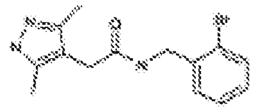
;

le *N*-[(4-chlorophényl)méthyl]-2-(3,5-diméthyl-1*H*-pyrazole-4-yl)acétamide (E7)

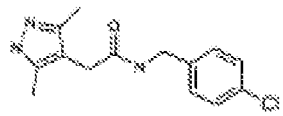
;

le *N*-[(2,4-dichlorophényl)méthyl]-2-(3,5-diméthyl-1*H*-pyrazole-4-yl)acétamide (E8)

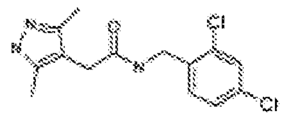
;

le *N*-[(3,4-dichlorophényl)méthyl]-2-(3,5-diméthyl-1*H*-pyrazole-4-yl)acétamide (E9)

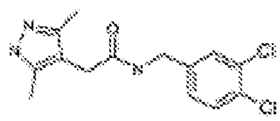
;

le *N*-[(3,5-dichlorophényl)méthyl]-2-(3,5-diméthyl-1*H*-pyrazole-4-yl)acétamide (E10)

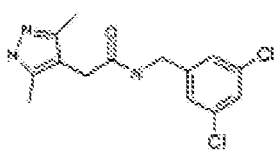
;

le *N*-[(2,6-dichlorophényl)méthyl]-2-(3,5-diméthyl-1*H*-pyrazole-4-yl)acétamide (E11)

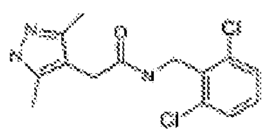
;

le *N*-[(2-chloro-6-méthylphényl)méthyl]-2-(3,5-diméthyl-1*H*-pyrazole-4-yl)acétamide (E12)

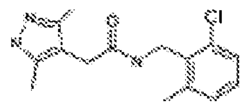 ;

le *N*-[(3-chlorophényl)méthyl]-2-(3,5-diméthyl-1*H*-pyrazole-4-yl)acétamide (E13)

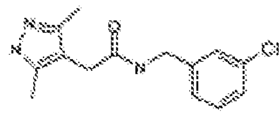 ;

le *N*-[(2-chloro-5-fluorophényl)méthyl]-2-(3,5-diméthyl-1*H*-pyrazole-4-yl)acétamide (E14)

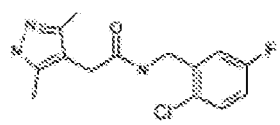 ;

le *N*-[(3-chloro-2-méthylphényl)méthyl]-2-(3,5-diméthyl-1*H*-pyrazole-4-yl)acétamide (E15)

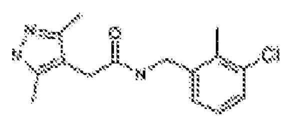 ;

le *N*-[(2-chloro-6-fluorophényl)méthyl]-2-(3,5-diméthyl-1*H*-pyrazole-4-yl)acétamide (E16)

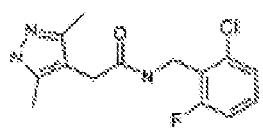 ;

le *N*-[(2,5-dichlorophényl)méthyl]-2-(3,5-diméthyl-1*H*-pyrazole-4-yl)acétamide (E17)

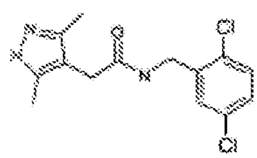 ;

le *N*-[(2-chlorophényl)méthyl]-2-(3,5-diméthyl-1*H*-pyrazole-4-yl)acétamide (E18)

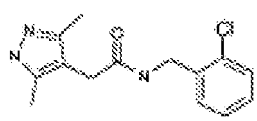 ;

le *N*-[(2,4-difluorophényl)méthyl]-2-(3,5-diméthyl-1*H*-pyrazole-4-yl)acétamide (E19)

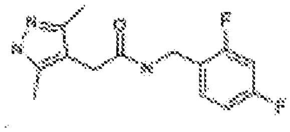;

le 2-(3,5-diméthyl-1*H*-pyrazole-4-yl)-*N*-{[4-fluoro-3-(trifluorométhyl)phényl]méthyl}acétamide (E20)

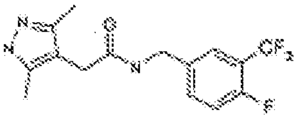;

le 2-(3,5-diméthyl-1*H*-pyrazole-4-yl)-*N*-{[4-fluoro-2-(trifluorométhyl)phényl]méthyl}acétamide (E21)

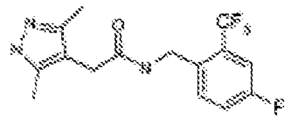;

le *N*-{[4-chloro-3-(trifluorométhyl)phényl]méthyl}-2-(3,5-diméthyl-1*H*-pyrazole-4-yl)acétamide (E22)

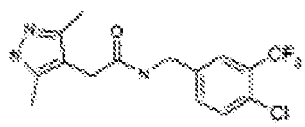;

le *N*-[(2,4-diméthylphényl)méthyl]-2-(3,5-diméthyl-1*H*-pyrazole-4-yl)acétamide (E23)

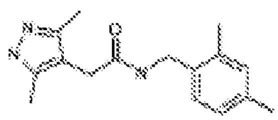;

le *N*-{[2-choro-3-(trifluorométhyl)phényl]méthyl}-2-(3,5-diméthyl-1*H*-pyrazole-4-yl)acétamide (E24)

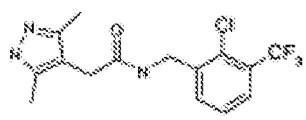;

le *N*-[(2-chloro-6-fluoro-3-méthylphényl)méthyl]-2-(3,5-diméthyl-1*H*-pyrazole-4-yl)acétamide (E25)

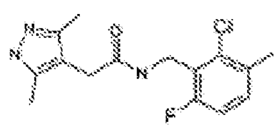;

le *N*-[(4-bromo-2-fluorophényl)méthyl]-2-(3,5-diméthyl-1*H*-pyrazole-4-yl)acétamide (E26)

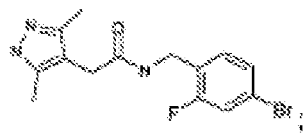

le *N*-[(6-chloro-2-fluoro-3-méthylphényl)méthyl]-2-(3,5-diméthyl-1*H*-pyrazole-4-yl)acétamide (E27)

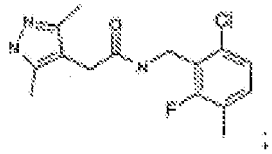

le 2-(3,5-diméthyl-1*H*-pyrazole-4-yl)-*N*-[(2-méthylphényl)-méthyl]acétamide (E28)

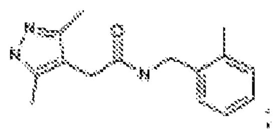

le 2-(3,5-diméthyl-1*H*-pyrazole-4-yl)-*N*-{[4-(trifluorométhyl)phényl]méthyl}acétamide (E29)

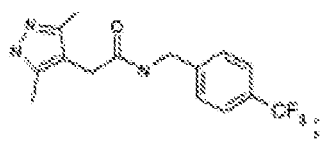

le *N*-[(2-chloro-4-fluorophényl)méthyl]-2-(3,5-diéthyl-1*H*-pyrazole-4-yl)acétamide (E30)

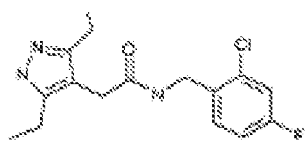

le 2-(3,5-diéthyl-1*H*-pyrazole-4-yl)-*N*-[(2,3,4-trifluorophényl)méthyl]acétamide (E31)

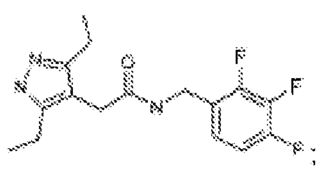

le 2-[3,5-bis(1-méthyléthyl)-1*H*-pyrazle-4-yl]-*N*-[(2-chloro-4-fluorophényl)méthyl]acétamide (E32)

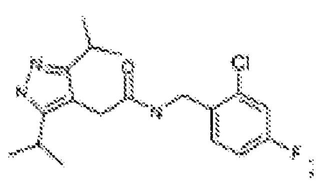

le 2-[3,5-bis(1-méthyléthyl)-1*H*-pyrazle-4-yl]-*N*-[(2,3,4-trifluorophényl)méthyl]acétamide (E33)

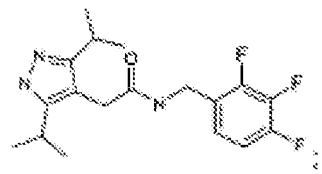

le *N*-[(2-chloro-4-fluorophényl)méthyl]-2-[3-méthyl-5-(trifluorométhyl)-1*H*-pyrazole-4-yl]acétamide (E34)

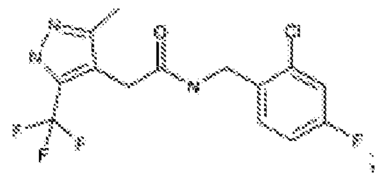

le *N*-[(2-chloro-3,4-difluorophényl)méthyl]-2-[3-méthyl-5-(trifluorométhyl)-1*H*-pyrazole-4-yl]acétamide (E35)

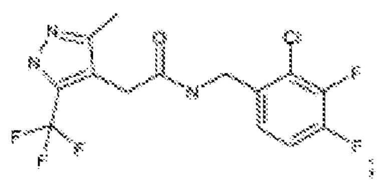

le *N*-{[2-chloro-3-(trifluorométhyl)phényl]méthyl}-2-[3-méthyl-5-(trifluorométhyl)-1*H*-pyrazole-4-yl]acétamide (E36)

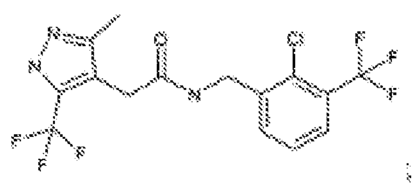

le 2-(3,5-diméthyl-1*H*-pyrazole-4-yl)-*N*-[1,2,3,4-tétrahydro-1-naphtalényl]acétamide (E37)

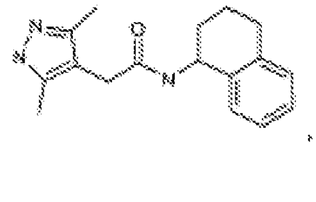

ou un de ses sels pharmaceutiquement acceptables.

**6.** Composition pharmaceutique qui comprend un composé de formule (I) ou un de ses sels pharmaceutiquement acceptables tel que défini dans l'une quelconque des revendications précédentes et un support ou un excipient pharmaceutiquement acceptable.

**7.** Composé, ou un de ses pharmaceutiquement acceptables, suivant l'une quelconque des revendications 1 à 5, destiné à être utilisé en thérapie.

**8.** Composé de formule (I) suivant l'une quelconque des revendications 1 à 5 ou un de ses sels pharmaceutiquement

acceptables, destiné à être utilisé dans le traitement d'un sujet humain ou animal souffrant de douleur, d'inflammation ou d'une maladie neurodégénérative.

**9.** Utilisation d'un composé, ou d'un de ses sels pharmaceutiquement acceptables, suivant l'une quelconque des revendications 1 à 5 pour la production d'un médicament destiné au traitement de la douleur, de l'inflammation ou d'une maladie neurodégénérative.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005019182 A
- US 4146721 A
- US 6133434 A


### Non-patent literature cited in the description


- **Collo et al.** *Neuropharmacology,* 1997, vol. 36, 1277-1283
- **Ferrari et al.** *J. Immunol.,* 2006, vol. 176, 3877-3883
- **Hide et al.** *Journal of Neurochemistry,* 2000, vol. 75, 965-972
- **Anderson, C. et al.** *Drug. Dev. Res.,* 2000, vol. 50, 92
- **Dell'Antonio et al.** *Neurosci. Lett.,* 2002, vol. 327, 87-90
- **Chessell, IP. et al.** *Pain,* 2005, vol. 114, 386-396
- **Honore et al.** *J. Pharmacol. Exp. Ther.,* 2006, vol. 319, 1376-1385
- **Skaper, S.D. et al.** *Glia,* 2006, vol. 54, 234-242
- **Parvathenani, L. et al.** *J. Biol. Chem.,* 2003, vol. 278 (15), 13309-13317
- **Berge ; Bighley ; Monkhouse.** *J. Pharm. Sci.,* 1977, vol. 66, 1-19
- **T.W. Greene ; P.G.M. Wuts.** Protective Groups in Organic Synthesis. J.Wiley and Sons, 1999
- The Chemistry of Acid Derivatives. **Ogliaruso, M.A. ; Wolfe, J.F.** The Chemistry of Functional Groups. John Wiley and Sons, 1979, 442-8
- The Chemistry of Amides. **Beckwith, A.L.J.** The Chemistry of Functional Groups. John Wiley and Sons, 1970, 73 ff